# EUROPEAN PATENT SPECIFICATION

(11) **EP 2 929 023 B1**
(45) Date of publication and mention of the grant of the patent: **16.08.2017**
(21) Application number: 13811687.6
(22) Date of filing: 02.12.2013
(51) Int. Cl.: C12N 9/24

(54) **COMPOSITIONS AND METHODS OF USE**
ZUSAMMENSETZUNGEN UND VERFAHREN ZUR VERWENDUNG
COMPOSITIONS ET PROCÉDÉS D'UTILISATION

(30) Priority: 07.12.2012 WO PCT/CN2012/086179
(43) Date of publication of application: 14.10.2015
(73) Proprietor: Danisco US Inc., Palo Alto, CA 94304 (US)
(72) Inventor: HUA, Ling, Palo Alto, CA 94304 (US); LAU, Rosalyn, Palo Alto, CA 94304 (US); LE, Steven, Palo Alto, CA 94304 (US); QIAN, Zhen, Palo Alto, CA 94304 (US); YU, Zheyong, Palo Alto, CA 94304 (US)
(74) Representative: Forrest, Graham Robert
(86) International application number: PCT/US2013/072584
(87) International publication number: WO 2014/088937

(56) References cited:
- WO-A2-2009/146464
- US-A- 5 631 144
- US-A1- 2006 154 843
- US-A1- 2011 053 218
- US-B2- 8 101 393
- A. LYKIDIS ET AL: "Genome Sequence and Analysis of the Soil Cellulolytic Actinomycete Thermobifida fusca YX", JOURNAL OF BACTERIOLOGY, vol. 189, no. 6, 15 March 2007 (2007-03-15), pages 2477-2486, XP55043253, ISSN: 0021-9193, DOI: 10.1128/JB.01899-06 & DATABASE UniProt [Online] 13 July 2005 (2005-07-13), Lykidis A. et al.: "Mannan endo-1,4-beta-mannosidase. Glycosyl Hydrolase family 5; EC=3.2.1.78", XP055103100, Database accession no. Q47RH9
- SUNIL S ADAV ET AL: "Label free quantitative proteomic analysis of secretome byon different lignocellulosic biomass", JOURNAL OF PROTEOMICS, ELSEVIER, AMSTERDAM, NL, vol. 75, no. 12, 22 April 2012 (2012-04-22), pages 3694-3706, XP028430304, ISSN: 1874-3919, DOI: 10.1016/J.JPROT.2012.04.031 [retrieved on 2012-05-02]
- E. BEKI ET AL: "Cloning and Heterologous Expression of a -D-Mannosidase (EC 3.2.1.25)-Encoding Gene from Thermobifida fusca TM51", APPLIED AND ENVIRONMENTAL MICROBIOLOGY, vol. 69, no. 4, 1 April 2003 (2003-04-01), pages 1944-1952, XP055103113, ISSN: 0099-2240, DOI: 10.1128/AEM.69.4.1944-1952.2003

## Description

### Technical Field

The present compositions and methods relates to a beta-mannanase derived from *Thermobifida fusca,* polynucleotides encoding the beta-mannanase, and methods for the production and use thereof. Formulations containing the recombinant beta-mannanase have a wide variety of uses, for instance, in hydrolyzing certain soft-wood type lignocellulosic materials and/or lignocellulosic biomass substrates comprising galactoglucomannan (GGM) and/or glucomannan (GM).

### Background

Cellulose and hemicellulose are the most abundant plant materials produced by photosynthesis. They can be degraded and used as an energy source by numerous microorganisms (*e*.*g*., bacteria, yeast and fungi) that produce extracellular enzymes capable of hydrolysis of the polymeric substrates to monomeric sugars (Aro et al., (2001) J. Biol. Chem., 276: 24309-24314). As the limits of non-renewable resources approach, the potential of cellulose to become a major renewable energy resource is enormous (Krishna et al., (2001) Bioresource Tech., 77: 193-196). The effective utilization of cellulose through biological processes is one approach to overcoming the shortage of foods, feeds, and fuels (Ohmiya et al. (1997) Biotechnol. Gen. Engineer Rev., 14: 365-414).

Most of the enzymatic hydrolysis of lignocellulosic biomass materials focus on cellulases, which are enzymes that hydrolyze cellulose (comprising beta-1,4-glucan or beta D-glucosidic linkages) resulting in the formation of glucose, cellobiose, cellooligosaccharides, and the like. Cellulases have been traditionally divided into three major classes: endoglucanases (EC 3.2.1.4) ("EG"), exoglucanases or cellobiohydrolases (EC 3.2.1.91) ("CBH") and beta-glucosidases ([beta]-D-glucoside glucohydrolase; EC 3.2.1.21) ("BG") (Knowles et al., (1987) TIBTECH 5: 255-261; and Schulein, (1988) Methods Enzymol., 160: 234-243). Endoglucanases act mainly on the amorphous parts of the cellulose fiber, whereas cellobiohydrolases are also able to degrade crystalline cellulose (Nevalainen and Penttila, (1995) Mycota, 303-319). Thus, the presence of a cellobiohydrolase in a cellulase system is required for efficient solubilization of crystalline cellulose (Suurnakki et al., (2000) Cellulose, 7: 189-209). Beta-glucosidase acts to liberate D-glucose units from cellobiose, cello-oligosaccharides, and other glucosides (Freer, (1993) J. Biol. Chem., 268: 9337-9342).

In order to obtain useful fermentable sugars from lignocellulosic biomass materials, however, the lignin will typically first need to be permeabilized, for example, by various pretreatment methods, and the hemicellulose disrupted to allow access to the cellulose by the cellulases. Hemicelluloses have a complex chemical structure and their main chains are composed of mannans, xylans and galactans. Mannan-type polysaccharides are found in a variety of plants and plant tissues, for example, in seeds, roots, bulbs and tubers of plants. Such saccharides may include mannans, galactomannas and glucomannans, and they typically containing linear and interspersed chains of linear beta-1,4-linked mannose units and/or galactose units. Most types of mannans are not soluble in water, forming the hardness characteristic of certain plant tissues like palm kernels and ivory nuts. Galactomannas, on the other hand, tend to be water soluble and are found in the seed endosperm of leguminous plants, and are thought to help with retention of water in those seeds.

Enzymatic hydrolysis of the complex lignocellulosic structure and rather recalcitrant plant cell walls involves the concerted and/or tandem actions of a number of different endo-acting and exo-acting enzymes *(e.g.,* cellulases and hemicellulases). Beta-xylanases and beta-mannanases are endo-acting enzymes, beta-mannosidase, beta-glucosidase and alpha-galactosidases are exo-acting enzymes. To disrupt the hemicellulose, xylanases together with other accessory proteins (non-limiting examples of which include L-α-arabinofuranosidases, feruloyl and acetylxylan esterases, glucuronidases, and β-xylosidases) can be applied.

Endo-1,4-beta-D-mannanases (E.C. 3.2.1.78) catalyzes the random hydrolysis of beta-1,4-mannosidic linkages in the main chain of mannan, galactomannanan, glucomannan, and galactoglucomannan, releasing short and long-chain oligomannosides. The short-chain oligomannosides may include mannobiose and mannotriose, although sometimes may also include some mannose. These can be further hydrolyzed by beta-mannosidases (E.C.3.2.1.25). In addition, the side-chain sugars of heteropolysaccharides can be further hydrolyzed, for example, to completion, by alpha galactosidase, beta-glucosidase, and/or by acetylmannan esterases. Puls J., (1997) Macromol. Symp. 120:183-196.

Beta-mannanases have been isolated from bacteria, fungi, plants and animals. *See,* Araujo A. et al., (1990) J. App. Bacteriol. 68:253-261; Dutta S. et al., (1997) Plant Physiol. 113:155-161; Puchar V. et al., (2004) Biochim. Biophys. Acta 1674:239-250. Genes encoding these enzymes from a number of organisms have also been cloned and sequenced, many if not all have been classified also as members of glycosyl hydrolase (GH) family 5 or 26, based on their sequences. *See, e.g.,* Bewley D.J., (1997) Planta 203:454-459; Halstead J.R. et al., (2000) FEMS Microl. Lett. 192:197-203; Xu B. et al., (2002) Eur. J. Biochem. 269:1753-1760; Henrissat, B. (1991) Biochem. J. 280:309-316. Although most beta-mannanases are secreted by the organisms from which they are originated, some are known to be associated with the cells. From a given organism there may be more than one mannanases with different isoelectric points derived from different genes or different products of the same genes, which fact is thought to be an indication of the importance of these enzymes.

Beta-mannanases have been used in commercially applications in, for example, industries such as the paper and pulp industry, foodstuff and feed industry, pharmaceutical industry and energy industry. Lee J.T., et al., (2003) Poult. Sci. 82:1925-1931; McCutchen M.C., et al., (1996) Biotechnol. Bioeng. 52:332-339; Suurnakki A., et al., (1997) Adv. Biochem. Eng. Biotechnol., 57:261-287. Depending on the microorganisms from which the mannanases are derived, however, different beta-mannanases may have different properties and activity profiles that may make them more suitable for one or more industrial applications but not for others. The hydrolysis of lignocellulosic biomass substrates, especially those from plant sources, is notoriously difficult, accordingly few if any mannanases that have been found to be useful in other industrial applications have been utilized to hydrolyze lignocellulosic materials.

Thus there exists a need to identify mannanases and/or compositions comprising such enzymes that are effective at and capable of, in conjunction with commercial, newly identified, or engineered cellulases and other hemicellulases, converting a wide variety of plant-based and/or other cellulosic or hemicellulosic materials into fermentable sugars with sufficient or improved efficacy, improved fermentable sugar yields, and/or improved capacity to act on a greater variety of cellulosic feedstock. The production of new mannanases using engineered microbes is also important and desirable because these are means through which enzymes can be cost-effectively made.

### Summary

The invention relates to the application or use of a highly active beta-mannanase (Tfu Man1) isolated from the bacterial species *Thermobifida fusca* strain YX, to hydrolyze a lignocellulosic biomass substrate. The herein described sequence of SEQ ID NO:2 was submitted to and published, as a part of a genomic sequence of Thermobifida fusca by Lykidis A. et al., to J. Bacteriol. (2007) 189 (6): 2477-86; and to the NCBI by Copeland et al., by a DOE Joint Genome Institute, which was designated with the Accession No. YP_288961. This sequence has been designated by the NCBI as a mannan endo-1,4-beta-mannosidase, but thus far it has not been recombinantly expressed, expressed in an industrially or commercially relevant amount, or applied in industrial applications. Tfu Man1 polypeptides have not been expressed by an engineered microorganism. Nor have they been coexpressed with, or included in a composition with, one or more cellulase genes and/or one or more hemicellulases.

The inventors have discovered that polypeptides having at least 65% (*e.g.,* at least 65%, at least 70%, at least 75%, at least 80%, at least 85%, at least 90%, at least 91%, at least 91%, at least 92%, at least 93%, at least 94%, at least 95%, at least 96%, at least 97%, at least 98%, at least 99% or higher) identity to SEQ ID NO:2, or to the mature sequence of SEQ ID NO:3, which is residues 29-453 of SEQ ID NO:2, have beta-mannanase activity. Further, when such a polypeptide is combined with one or more cellulases it confers improved capacity of that composition or mixture to hydrolyze lignocellulosic biomass substrates. Such improvements include, for example, one or more of the properties selected from: an increased glucan conversion, an increased glucose yield from a given biomass substrate, an increased xylan conversion, an increased xylose yield, an increased total soluble sugar yield from a given biomass substrate, a more rapid liquefaction of a given biomass substrate at a solids level, and a more rapid viscosity reduction of a biomass substrate at a solids level. Improvements also may include the surprising finding that such a polypeptide can be used to boost the cellulosic biomass conversion and hydrolysis when in combination with a cellulase mixture or composition, which optionally further comprises one or more other hemicellulase. The resulting mixture comprising the Tfu Man1 polypeptide has improved hydrolysis performance as compared to a counterpart mixture having all the other enzymes at the same concentrations/proportion/amounts, but without the Tfu Man1. In some embodiments, the Tfu Man1 polypeptides can substitute, for example, for up to about 20 wt.% *(e.g.,* up to about 20 wt.%, up to about 18 wt.%, up to about 16 wt.%, up to about 14 wt.%, up to about 12 wt.%, up to about 10 wt.%, up to about 8 wt.%, up to about 5 wt.%, etc) of a cellulase mixture or composition, and the substituted composition when used to hydrolyze a given lignocellulosic biomass substrate will retain its capacity and hydrolysis performance, or even have improved hydrolysis *(e.g.,* higher glucan and/or xylan conversion, higher production of total sugars, faster liquefaction, and/or improved viscosity reduction) than a un-substituted counterpart cellulase mixture or composition of otherwise the same enzyme composition and the same total protein.
The invention provides enzyme compositions and methods utilizing said compositions as set out in the claims.

An aspect of the present composition and methods pertains to a beta-mannanase polypeptide of glycosyl hydrolase family 5 derived from *Thermobifida fusca,* referred to herein as "Tfu Man1" or a "Tfu Man1 polypeptide," compositions comprising the same, and methods of producing and applying the compositions comprising thereof in hydrolyzing or converting lignocellulosic biomass into soluble, fermentable sugars. Particularly suitable lignocellulosic biomass materials are those that contain galactoglucomannan (GGM) and/or glucomannan (GM). Such fermentable sugars can then be converted into cellulosic ethanol, fuels, and other biochemicals and useful products. In certain embodiments, the beta-mannanase polypeptides, when combined with an enzyme mixture comprising at least one cellulase, or with an enzyme mixture comprising at least one cellulase and at least one other hemicellulase, resulted in an enzyme mixture that is capable of increased or enhanced capacity to hydrolyze a lignocellulosic biomass material, as compared to, for example, other beta-mannanases from various microbes, which have similar pH optimum and/or similar temperature optimum.

Such increased or enhanced capacity to hydrolyze a lignocellulosic biomass material is reflected, for example, in substantially increased production of not only total soluble sugars, but surprisingly also increased production of glucose (reflecting a higher glucan conversion) and/or increased production of xylose (reflecting a higher xylan conversion), produced by enzymatic hydrolysis of a given lignocellulosic biomass substrate pretreated in a certain way.

The increased or enhanced capacity to hydrolyze a lignocellulosic biomass material can also be reflected in the desirable capacity of such an enzyme composition to improve or accelerate liquefaction and/or reduce viscosity of the pretreated biomass material. Such a viscosity/ liquefaction benefit is the most prominent if a high solids level of the biomass material is used as a substrate. The viscosity/liquefaction benefits are also substantial and important when the enzyme composition/mixture is used to break down or hydrolyze a woody biomass, which tends to be highly fibrous and recalcitrant, making for particularly viscous feedstocks.

The increased or enhanced capacity to hydrolyze a lignocellulosic biomass allows the substitution of up to about 20 wt.% (e.g., up to about 20 wt.%, up to about 18 wt.%, up to about 16 wt.%, up to about 14 wt.%, up to about 12 wt.%, up to about 10 wt.%, up to about 8 wt.%, up to about 5 wt.%, etc) of any given cellulase composition, which optionally comprises one or more other hemicellulases, with a Tfu Man1 polypeptide, thereby reducing the amount of cellulase composition and the enzymes therein used to hydrolyze a given substrate without sacrificing performance. Indeed, the hydrolysis performance may even be improved using the substituted composition. Reducing the amount of cellulase composition as well as the amount of enzymes therein required to hydrolyze or saccharify a lignocellulosic biomass result substantial cost-savings to produce a cellulosic sugar, which can then be made into ethanol or other down-stream valuable bio-chemicals and useful products.

Aspects of the present compositions and methods are drawn to beta-mannanase derived from *Thermobifida fusca,* referred to herein as "Tfu Man1" or "Tfu Man1 polypeptides," and methods of employing the beta-mannanase in various industrially useful applications, for example, in hydrolyzing or converting lignocellulosic biomass into soluble, fermentable sugars. Such fermentable sugars can then be converted into cellulosic ethanol, fuels, and other bio-chemicals and useful products. As demonstrated herein, Tfu Man1 polypeptides as well as compositions comprising Tfu Man1 polypeptides have improved performance, when combined with at least one cellulase and optionally also at least one other hemicellulase, in hydrolyzing lignocellulosic biomass substrates, especially those that contain at least some measurable levels of galactoglucomannan (GGM) and/or glucomannan (GM), as compared to other beta-mannanases from similar microorganisms having similar pH optimums and/or temperature optimums. The improved performance may be that the Tfu Man1 polypeptides and/or enzyme compositions comprising Tfu Man1 polypeptides produces increased amounts of total soluble sugars when used to hydrolyze a lignocellulosic biomass substrate, under suitable conditions for the enzymatic hydrolysis, when compared to other microbial beta-mannanases having similar pH optimums and/or temperature optimums. Surprisingly the Tfu Man1 polypeptides and/or the compositions comprising such polypeptides also have improved glucan conversion and/or improved xylan conversion, as compared to those other microbial beta-mannanases having similar pH optimums and/or temperature optimums. The improved performance may alternatively or also be that the Tfu Man1 polypeptides and/or enzyme compositions comprising Tfu Man1 polypeptides confer rapid viscosity reduction /liquefaction to the biomass substrate, such that the overall hydrolysis is improved in not only effectiveness but also efficiency.

Tfu Man1 polypeptide is applied together with, or in the presence of, one or more cellulases in an enzyme composition to hydrolyze or breakdown a suitable biomass substrate. The one or more cellulases may be, for example, one or more beta-glucosidases, cellobiohydrolases, and/or endoglucanases. For example, the enzyme composition may comprise a Tfu Man1 polypeptide, a beta-glucosidase, a cellobiohydrolase, and an endoglucanase. In some embodiments, at least one of the cellulases is heterologous to the Tfu Man1, in that at least one of the cellulases is not derived from a *Thermobifida fusca.* In some embodiments, at least two among the cellulases are heterologous from each other.

In some embodiments, a Tfu Man1 polypeptide is applied together with, or in the presence of one or more cellulases and one or more hemicellulases in an enzyme composition. The one or more other hemicellulases may be, for example, other mannanases, xylanases, beta-xylosidases, and/or L-arabinofuranosidases. In some embodiments, at least one of the other hemicellulases is heterologous to the Tfu Man1, in that at least one of the other hemicellulases, which may be selected from one or more other mannanases, xylanases, beta-xylosidases, and/or L-arabinofuranosidases, is not derived from a *Thermobifida fusca.* In certain embodiments, at least two of the other hemicellulases are heterologous to each other.

For example, the enzyme composition comprises a Tfu Man1 polypeptide, no or one or two other mannanases, one or more cellobiohydrolases, one or more endoglucanases, one or more beta-glucosidases, no or one or more xylanases, no or one or more beta-xylosidases, and no or one or more L-arabinofuranosidases.

In some embodiments, a Tfu Man1 polypeptide is used to substitute up to about 20 wt.% (based on total weight of proteins in a composition) *(e.g.,* up to about 20 wt.%, up to about 18 wt.%, up to about 16 wt.%, up to about 14 wt.%, up to about 12 wt.%, up to about 10 wt.%, up to about 8 wt.%, up to about 5 wt.%, etc) of an enzyme composition comprising one or more cellulases, optionally also one or more other non-Tfu Man1 hemicellulases. In some embodiments, the thus-substituted enzyme composition has similar or improved saccharification performance as the counterpart unsubstituted enzyme composition having no Tfu Man1 present but all the other cellulases and hemicellulases, as well as the same total weight of proteins in the composition. In some embodiments, the substituted enzyme composition can produce the same amount of glucose and/or xylose, or an about 5% higher amount of glucose and/or xylose, about 7% higher amount of glucose and/or xylose, about 10% higher amount of glucose and/or xylose, or an even greater amount of glucose and/or xylose from the same lignocellulosic biomass substrate, as compared to the un-substituted counterpart enzyme composition having no Tfu Man1 but all the other cellulases and hemicellulases, and comprising the same total weight of proteins in the composition. In some embodiments, when used to hydrolyze a given lignocellulosic biomass substrate at a given solids level, the substituted enzyme composition reduces the viscosity of the biomass substrate by the same extent or to a higher extent, when compared to the un-substituted counterpart enzyme composition comprising no Tfu Man1 but all the other cellulases and hemicellulases, and comprising the same total weight of proteins in the composition.

In certain embodiments, a Tfu Man1 polypeptide, or a composition comprising the Tfu Man1 polypeptide is applied to a lignocellulosic biomass substrate or a partially hydrolyzed lignocellulosic biomass substrate in the presence of an ethanologen microbe, which is capable of metabolizing the soluble fermentable sugars produced by the enzymatic hydrolysis of the lignocellulosic biomass substrate, and converting such sugars into ethanol, biochemicals or other useful materials. Such a process may be a strictly sequential process whereby the hydrolysis step occurs before the fermentation step. Such a process may, alternatively, be a hybrid process, whereby the hydrolysis step starts first but for a period overlaps the fermentation step, which starts later. Such a process may, in a further alternative, be a simultaneous hydrolysis and fermentation process, whereby the enzymatic hydrolysis of the biomass substrate occurs while the sugars produced from the enzymatic hydrolysis are fermented by the ethanologen.

The Tfu Man1 polypeptide, for example, may be a part of an enzyme composition, which is a whole broth product of an engineered microbe capable of expressing or over-expressing such a polypeptide under suitable conditions. In certain embodiments, the Tfu Man1 polypeptide may be genetically engineered to express in a bacterial host cell, for example, in *Escherichia, Bacillus, Lactobacillus, Pseudomonas,* or *Streptomyces.* In certain embodiments, the Tfu Man1 polypeptide may be genetically engineered to express in a fungal host cell, for example, in a host cell of any one of the filamentous forms of the subdivision *Eumycotina.* Thus suitable filamentous fungal host cells may include, without limitation, cells of *Acremonium, Aspergillus, Aureobasidium, Bjerkandera, Ceriporiopsis, Chrysoporium, Coprinus, Coriolus, Corynascus, Chaertomium, Cryptococcus, Filobasidium, Fusarium, Gibberella, Humicola, Maguaporthe, Mucor, Myceliophthora, Mucor, Neocallimastix, Neurospora, Paecilomyces, Penicillium, Phanerochaete, Phlebia, Piromyces, Pleurotus,Scytaldium, Schizophyllum, Sporotrichum, Talaromyces, Thermoascus, Thielavia, Tolypocladium, Trametes,* and *Trichoderma.*

The engineered microbe expressing or over-expressing the Tfu Man1 polypeptide may also express and/or secrete one or more or all of one or more cellulases and optionally also one or more other hemicellulases. The one or more cellulases may be selected from, for example, one or more endoglucanases, one or more beta-glucosidases, and/or one or more cellobiohydrolases. The one or more other hemicellulases may be selected from, for example, one or more other beta-mannanases, one or more Alpha-L-arabinofuranosidases, one or more xylanases, and/or one or more beta-xylosidases. The resulting enzyme mixture comprising the Tfu Man1 polypeptide is a "co-expressed enzyme mixture" for the purpose of this application.

In another embodiment, the engineered microbe expressing or over-expressing the Tfu Man1 polypetpide may be one that is different from the one or more other microbes expressing one or more of the cellulases and optionally also one or more of the other hemicellulases. The one or more cellulases may be selected from, for example, one or more endoglucanases, one or more beta-glucosidases, and/or one or more cellobiohydrolases. The one or more other hemicellulases may be selected from, for example, one or more other beta-mannanases, one or more Alpha-L-arabinofuranosidases, one or more xylanases, and/or one or more beta-xylosidases. Accordingly the Tfu Man1 polypeptide can be combined with one or more cellulases and optionally also one or more other hemicellulases to form an enzyme mixture/composition, which is a "physical mixture" or "admixture" of Tfu Man1 and other polypeptides. The improved capacity observable or achievable with the co-expressed enzyme mixture is also observable or achievable with the admixture comprising Tfu Man1.

As demonstrated herein, Tfu Man1 polypeptides and compositions comprising Tfu Man1 polypeptides have improved efficacy at conditions under which saccharification and degradation of lignocellulosic biomass take place. The improved efficacy of an enzyme composition comprising a Tfu Man1 polypeptide is shown when its performance of hydrolyzing a given biomass substrate is compared to that of an otherwise comparable enzyme composition comprising certain other microbial beta-mannanases having similar pH optimums and/or temperature optimums. In certain embodiments, Tfu Man1 polypeptides of the compositions and methods herein have at least about 5 % (for example, at least about 5%, at least about 7%, at least about 10%, at least about 12%, at least about 13%, at least about 14%, at least about 15%, or more) increased capacity to hydrolyze a given lignocellulosic biomass substrate, which has optionally been subject to pretreatment, as compared to a ScoMan1 polypeptide from *Streptomyces coelicolorA3,* comprising the amino acid sequence of SEQ ID NO:4, or Bsp Man1 polypeptide from *Bacillus caldovelox,* comprising the amino acid sequence of SEQ ID NO:5, or Msp Man2 polypeptide from *Micromonospora sp. L5,* comprising the amino acid sequence of SEQ ID NO:6. The performance of hydrolyzing a given biomass substrate can be measured using the amount of total soluble sugars produced from a given lignocellulosic biomass under a given set of saccharification conditions. The performance of hydrolyzing a given biomass substrate can also be measured using the amount of glucose produced from a given lignocellulosic biomass substrate under a saccharification condition or the % glucan conversion from that biomass substrate. For example, % glucan conversion can be assessed using a method described in Example 9 (herein). As such, a Tfu Man1 polypeptide of the compositions and methods herein, when included in a given enzyme composition in a certain amount, confers at least a 5% increase (for example, a 5% increase, a 7% increase, a 10% increase, a 11% increase, a 12% increase, a 13% increase, a 14% increase, a 15% increase, or a higher percent increase) in % glucan conversion when it is a part of an enzyme composition as compared to the otherwise same enzyme composition comprising the same amount of ScoMan1, or the same amount of Bsp Man1, or the same amount of Msp Man2, under the same hydrolysis conditions. Alternatively or in addition, the performance of hydrolyzing a given biomass substrate can be measured using the amount of xylose produced from a given lignocellulosic biomass substrate under a saccharification condition or the % xylan conversion from that substrate. For example % xylan conversion can be assessed using a method described in Example 9 (herein). As such, a Tfu Man1 polypeptide of the compositions and methods herein, when included in a given enzyme composition in a certain amount, confers at least a 5% increase (for example, a 5% increase, a 7% increase, a 10% increase, a 11% increase, a 12% increase, a 13% increase, a 14% increase, a 15% increase, or a higher percent increase) in % xylan conversion when it is a part of an enzyme composition as compared to the otherwise same enzyme composition comprising the same amount of ScoMan1, or the same amount of Bsp Man1, or the same amount of Msp Man2, under the same hydrolysis conditions.

Furthermore, the performance of hydrolyzing a given biomass substrate can be measured by the extent or degree of liquefaction or viscosity reduction of the biomass substrate or the speed of such liquefaction or viscosity reduction of a given substrate having a particular solids level. The viscosity reduction and/or liquefaction and the rate thereof can be assessed using a method described in Example 10 (herein). As such a Tfu Man1 polypeptide of the compositions and methods herein, when included in a given enzyme composition in a certain amount, confers at least a 5% higher viscosity reduction or level of liquefaction as compared to an otherwise same enzyme composition comprising the same amount of ScoMan1, or the same amount of Bsp Man1, or the same amount of Msp Man2, under the same hydrolysis conditions and after the hydrolysis reaction is carried on for the same time period.

The present compositions and methods employ a recombinant polypeptide comprising an amino acid sequence that is at least 65% identical to the amino acid sequence of SEQ ID NO:2 or SEQ ID NO:3, wherein the polypeptide has beta-mannanase activity. In some aspects, a Tfu Man1 polypeptide as it is applied in an enzyme composition or in a method to hydrolyze a lignocellulosic biomass substrate is (a) derived from, obtainable from, or produced by *Thermobifida fusca,* for example, an actinobacterium *Thermobifida fusca* strain YX; (b) a recombinant polypeptide comprising an amino acid sequence that is at least 65% *(e.g.,* at least 65%, 70%, 75%, 80%, 85%, 90%, 91%, 92%, 93%, 94%, 95%, 96%, 97%, 98%, 99%, or 100%) identical to the amino acid sequence of SEQ ID NO:2; (c) a recombinant polypeptide comprising an amino acid sequence that is at least 65% *(e.g.,* at least 65%, 70%, 75%, 80%, 85%, 90%, 91%, 92%, 93%, 94%, 95%, 96%, 97%, 98%, 99%, or 100%) identical to the catalytic domain of SEQ ID NO:2, namely amino acid residues 29 to 453; (d) a recombinant polypeptide comprising an amino acid sequence that is at least 65% (e.g., at least 65%, 70%, 75%, 80%, 85%, 90%, 91%, 92%, 93%, 94%, 95%, 96%, 97%, 98%, 99%, or 100%) identical to the mature form of amino acid sequence of SEQ ID NO:3, namely amino acid residues 29-453 of SEQ ID NO:2; or (e) a fragment of (a), (b), (c) or (d) having beta-mannanase activity. In certain embodiments, it is provided a variant polypeptide having beta-mannanase activity, which comprises a substitution, a deletion and/or an insertion of one or more amino acid residues of SEQ ID NO:2 or SEQ ID NO:3. In certain embodiments, the polypeptide comprises an amino acid sequence that is at least 80% identical to the amino acid sequence of SEQ ID NO:2 or SEQ ID NO: 3. In certain embodiments, the polypeptide comprises an amino acid sequence that is at least 90% identical to the amino acid sequence of SEQ ID NO:2 or SEQ ID NO: 3. In certain embodiments, the polypeptide comprises an amino acid sequence that is at least 95% identical to the amino acid sequence of SEQ ID NO:2 or SEQ ID NO: 3. In certain embodiments, the polypeptide comprises an amino acid sequence that is at least 99% identical to the amino acid sequence of SEQ ID NO:2 or SEQ ID NO: 3.

In certain embodiments, the Tfu Man1 polypeptide has a pH optimum of about pH 5.0. The Tfu Man1 polypeptide retains greater than 70% of its maximum activity between pH 4.2 and pH 7.8.

In certain embodiments, the Tfu Man1 polypeptide has an optimum temperature of about 71°C. The Tfu Man1 polypeptide retains greater than 80% of its maximum activity between the temperatures of 58°C and 78°C.

In certain embodiments, the Tfu Man1 polypeptide has good thermostability. For example, the Tfu Man1 polypeptide retains about 50% of the beta-mannanase activity when incubated for about 2 hours at a temperature of about 72°C. In certain embodiments, the polyeptpides retains at least 99% of the beta-mannanase activity when incubated for about 2 hours at a temperature of lower than 60°C.

Aspects of the present compositions and methods include a composition comprising the recombinant Tfu Man1 polypeptide as described herein and one or more cellulases. In some embodiments, the one or more cellulases may be selected from one or more endoglucanases, one or more cellobiohydrolases and/or one or more beta-glucosidases.

Aspects of the present compositions and methods include a composition comprising the recombinant Tfu Man1 polypeptide as described herein and one or more cellulases and one or more other hemicellulases. For example, the one or more cellulases may be selected from endoglucanases, cellobiohydrolases, and/or beta-glucosidases, and the one or more other hemicellulases may include xylanases, beta-xylosidases, alpha-L-arabinofuranosidases and other mannanases.

As demonstrated herein, the Tfu Man1 polypeptides described herein can impart, to an enzyme mixture or composition comprising a Tfu Man1 polypeptide in addition to one or more cellulases, an improved capacity to hydrolyze, saccharify, or degrade a given lignocellulosic biomass substrate, which has optionally been subject to pretreatment, and further optionally having had at least some of its xylan-containing components removed or separated from the glucan-containing components. Such improved capacity to hydrolyze, saccharify, or degrade a given lignocellulosic biomass substrate may be evidenced by a measurably higher %glucan conversion achieved using a given enzyme composition comprising at least one cellulase, and a Tfu Man1 polypeptide in an amount of as high as about 20 wt.% (for example, up to about 2 wt.%, up to about 5 wt.%, up to about 7 wt.%, up to about 10 wt.%, up to about 12 wt.%, up to about 15 wt.%, up to about 16 wt.%, up to about 17 wt.%, up to about 18 wt.%, up to about 19 wt.%, up to about 20 wt.%) of the enzyme composition, to hydrolyze a particular lignocellulosic biomass substrate, as compared to a counterpart enzyme composition comprising all the same other enzymes in the same proportion but comprising no Tfu Man1 polypeptide.

Aspects of the present compositions and methods include a composition comprising a recombinant Tfu Man1 polypeptide as detailed herein and a lignocellulosic biomass. Suitable lignocellulosic biomass may be, for example, derived from an agricultural crop, a byproduct of a food or feed production, a lignocellulosic waste product, a plant residue, including, for example, a grass residue, or a waste paper or waste paper product. Certain particularly suitable biomass may be one that comprises at least a measurable level of galactoglucomannan (GGM) and/or glucomannan (GM). Suitably the biomass may preferably be one that is rich in galactoglucomannan (GGM) and/or in glucomannan (GM), for example one that comprises at least about 0.5 wt.% (e.g., 0.5 wt.%, at least about 0.7 wt.%, at least about 1.0 wt.%, at least about 1.2 wt.%, at least about 1.5 wt.%, at least about 2.0 wt.%, at least about 2.5 wt.%, or more) GGM, or at least about 0.5 wt.% (e.g., 0.5 wt.%, at least about 0.7 wt.%, at least about 1.0 wt.%, at least about 1.2 wt.%, at least about 1.5 wt.%, at least about 2.0 wt.%, at least about 2.5 wt.%, or more) GM, or at least about 0.5 wt.% (e.g., 0.5 wt.%, at least about 0.7 wt.%, at least about 1.0 wt.%, at least about 1.2 wt.%, at least about 1.5 wt.%, at least about 2.0 wt.%, at least about 2.5 wt.%, at least about 3.0 wt.%, at least about 3.5 wt.%, at least about 4.0 wt.%, at least about 4.5 wt.%, at least about 5.0 wt.%, or more) of GGM and GM combined. In certain embodiments, the lignocellulosic biomass has been subject to one or more pretreatment steps in order to render xylan, hemicelluloses, cellulose and/or lignin material more accessible or susceptible to enzymes and thus more amendable to enzymatic hydrolysis. A suitable pretreatment method may be, for example, subjecting biomass material to a catalyst comprising a dilute solution of a strong acid and a metal salt in a reactor. *See, e.g.,* U.S. Patent Nos. 6,660,506, 6,423,145. Alternatively, a suitable pretreatment may be, for example, a multi-stepped process as described in U.S. Patent No. 5,536,325. In certain embodiments, the biomass material may be subject to one or more stages of dilute acid hydrolysis using about 0.4% to about 2% of a strong acid, in accordance with the disclosures of U.S. Patent No. 6,409,841. Further embodiments of pretreatment methods may include those described in, for example, U.S. Patent No. 5,705,369; in Gould, (1984) Biotech. & Bioengr., 26:46-52; in Teixeira et al., (1999) Appl. Biochem & Biotech., 77-79:19-34; in International Published Patent Application WO2004/081185; or in U.S. Patent Publication No. 20070031918, or International Published Patent Application WO06110901. A non-limiting example of a suitable lignocellulosic biomass substrate is a softwood substrated pretreated using the US Department of Agriculture's SPORL protocol, as described in Example 10 herein. Another non-limiting example of a suitable lignocellulosic biomass substrate is an akaline KRAFT-pretreated softwood pulp FPP-27.

In further aspects, the present invention pertains to a method of applying or using the composition as described above under conditions suitable for degrading or converting a cellulosic material and for producing a substance from a cellulosic material.

In a further aspect, methods for degrading or converting a cellulosic material into fermentable sugars are provided, comprising: contacting the cellulosic material, preferably having already been subject to one or more pretreatment steps, with the Tfu Man1 polypeptides or the compositions comprising such polypeptides of one of the preceding paragraphs to yield fermentable sugars.

### Brief Description of the Drawings

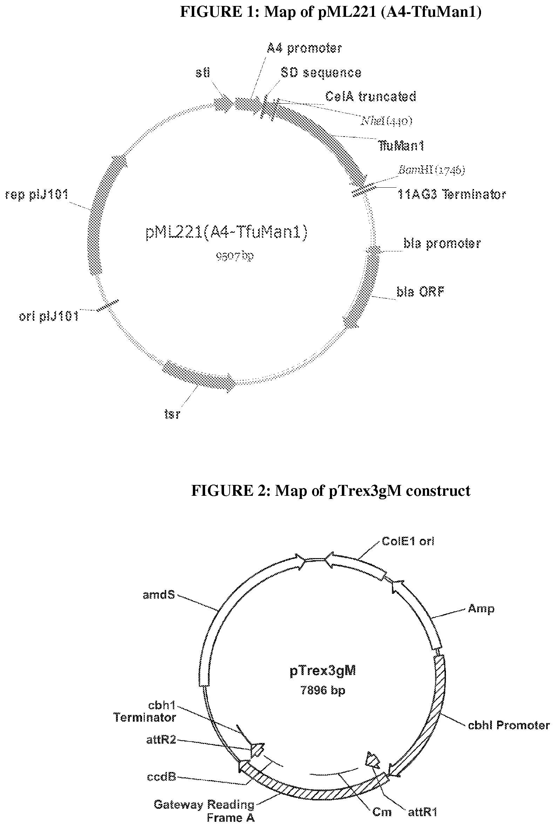

**Figure 1** depicts a map of the pML221(A4-TfuMan1) vector.
**Figure 2** depicts a map of the pTrex3gM construct.

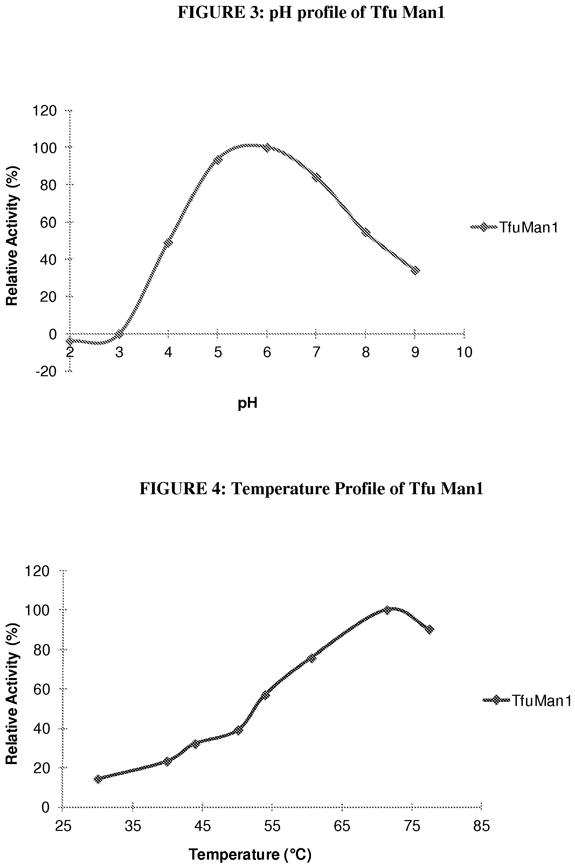

**Figure 3** depicts a pH profile of Tfu Man1. The effect of pH on beta-mannanase activity of Tfu Man1 was measured at 50°C for 10 minutes using 1% locust bean gum as substrate in 50 mM sodium citrate and 50 mM sodium phosphate buffer adjusted to individual pH values ranging between pH 2-9. The mannanase activity of the Tfu Man1 polypeptide at its pH optimum was normalized to 100%, and the mannanase activity of the same polypeptide at other pH values were depicted as relative activity to that at the pH optimum.
**Figure 4** depicts a temperature profile of Tfu Man 1. The effect of temperature change on beta-mannanase activity of Tfu Man1 was measured at individual temperature values ranging between 40°C and 84°C for 10 minutes using 1% locust bean gum as substrate in a 50 mM sodium citrate buffer, at pH 6.0. The mannanase activity of the Tfu Man1 polypeptide at its temperature optimum was normalized to 100%, and the mannanase activity of the same polypeptide at other temperature values were depicted as relative activity to that at the temperature optimum.

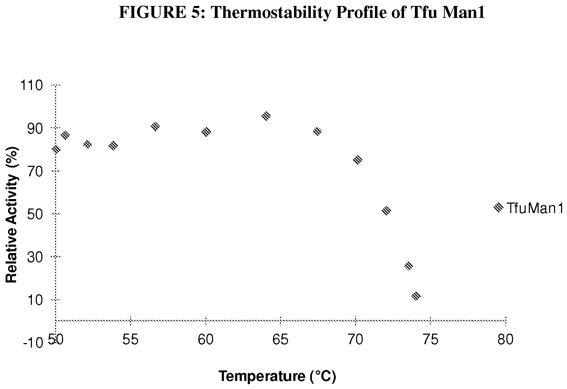

**Figure 5** depicts a thermostability profile of Tfu Man1. The thermostability of Tfu Man1 was determined by incubation in 50 mM sodium citrate buffer at pH 6.0 at a set temperature within the range of 40°C and 65°C for 2 hours. After incubation, the remaining mannanase activity at each of the incubation temperature was measured. The activity measured from a control sample of Tfu Man1 polypeptide kept on ice for the same 2 hours was used as the 100% activity to normalize the residual activity measurements.

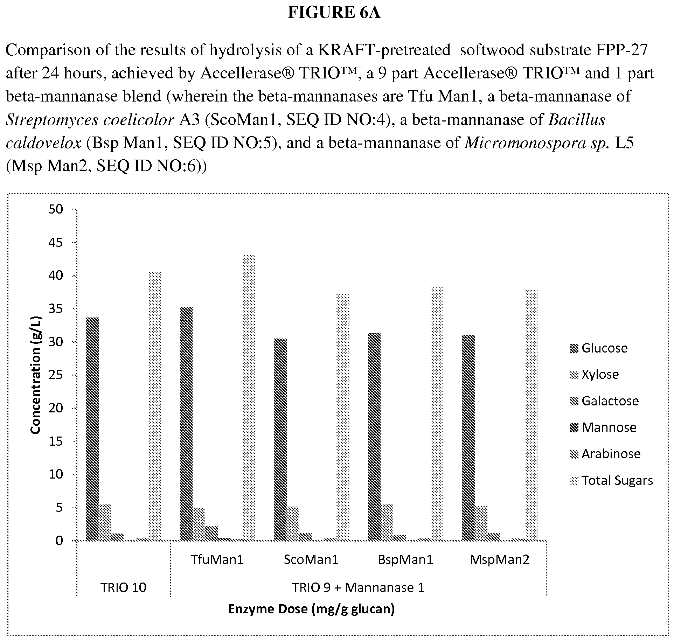

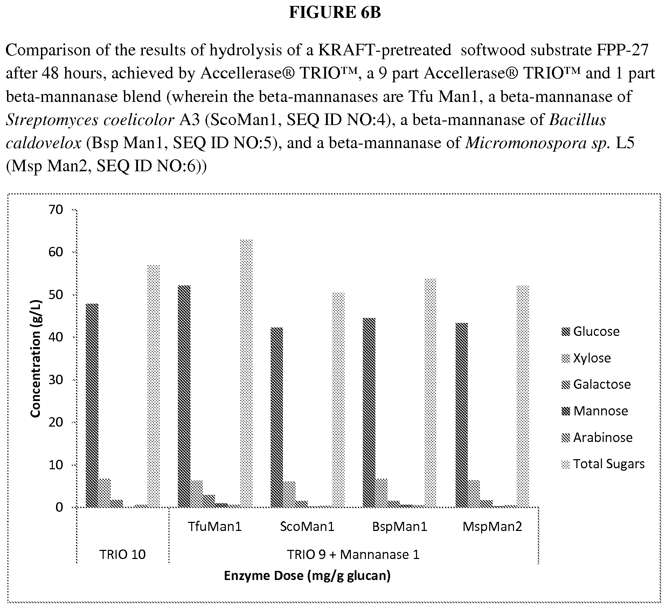

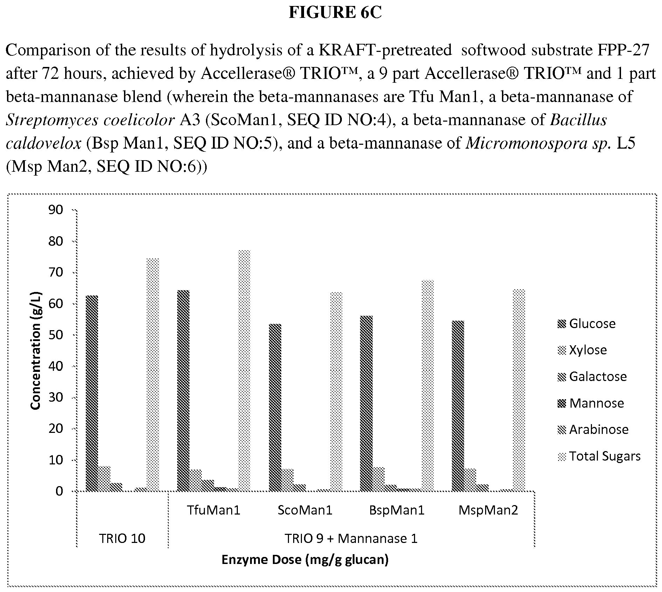

**Figures 6A-6C** depict the comparison of levels of hydrolysis achieved by a commercial cellulase/hemicellulase composition Accellerase® TRIO™ vs. a blend of 9 parts Accellerase® TRIO™ with 1 part *(i.e.,* 10 wt.%) of a Tfu Man1 polypeptide, as compared to the same blend of Accellerase® TRIO™ with each of three other beta-mannanases of GH5, a *Streptomyces coelicolor A3* beta-mannanase of SEQ ID NO:4 ("ScoMan1"), a *Bacillus caldovelox* beta-mannanase of SEQ ID NO:5 ("Bsp Man1"), and a *Micromonospora sp. L5* beta-mannanase of SEQ ID NO:6 ("Msp Man2"), of a given biomass substrate, namely the alkaline KRAFT- pretreated softwood substrate FPP-27, under the same hydrolysis conditions and at different durations of reaction. **Figure 6A** depicts the results of hydrolysis after 24 hours. **Figure 6B** depicts the results of hydrolysis after 48 hours. **Figure 6C** depicts the results of hydrolysis after 72 hours. Details of the experiments are found in Example 9.

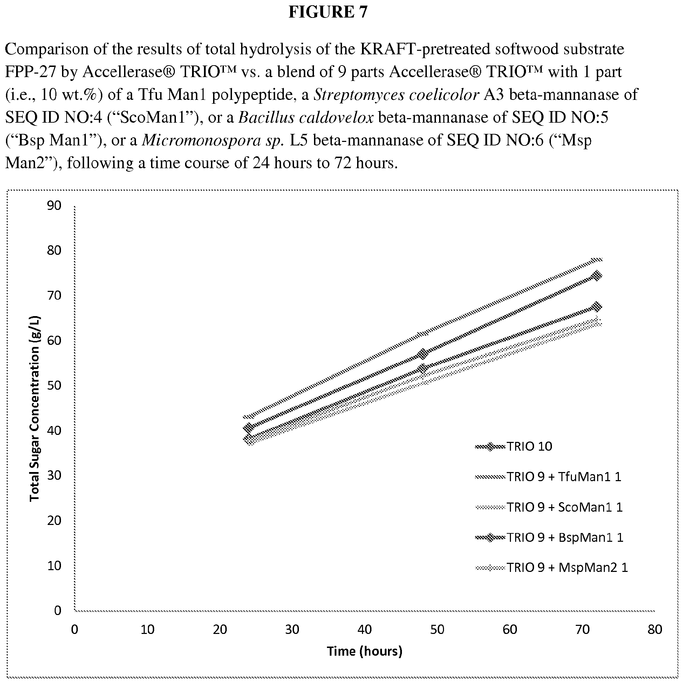

**Figure 7** depicts the comparison of total hydrolysis of the FPP-27 alkaline KRAFT- pretreated softwood substrate by Accellerase® TRIO™ vs. a blend of 9 parts Accellerase® TRIO™ with 1 part *(i.e.,* 10 wt.%) of a Tfu Man1 polypeptide, a *Streptomyces coelicolorA3* beta-mannanase of SEQ ID NO:4 ("ScoMan1"), a *Bacillus caldovelox* beta-mannanase of SEQ ID NO:5 ("Bsp Man1"), and a *Micromonospora sp. L5* beta-mannanase of SEQ ID NO:6 ("Msp Man2"), following a time course of 24 hours to 72 hours. Details of the experiments are found in Example 9.

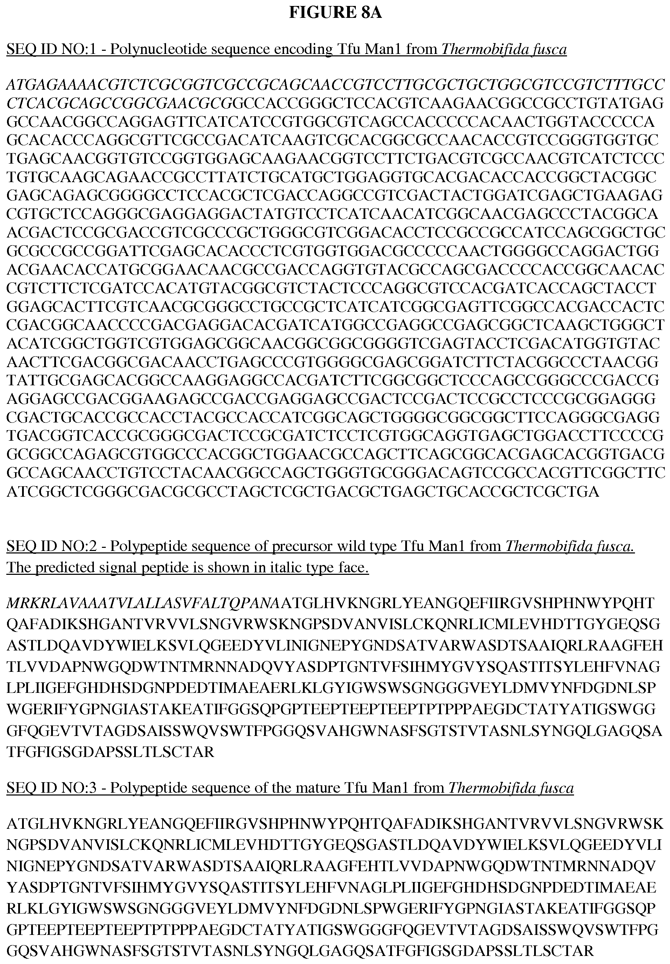

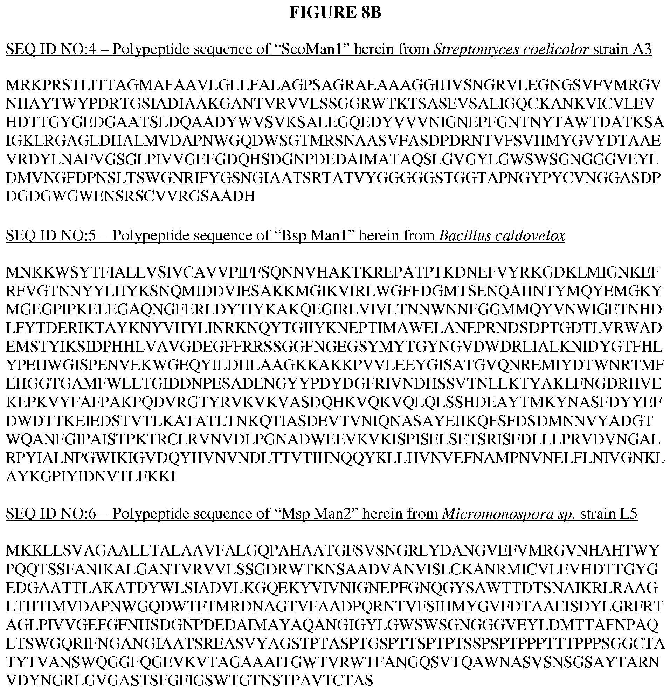

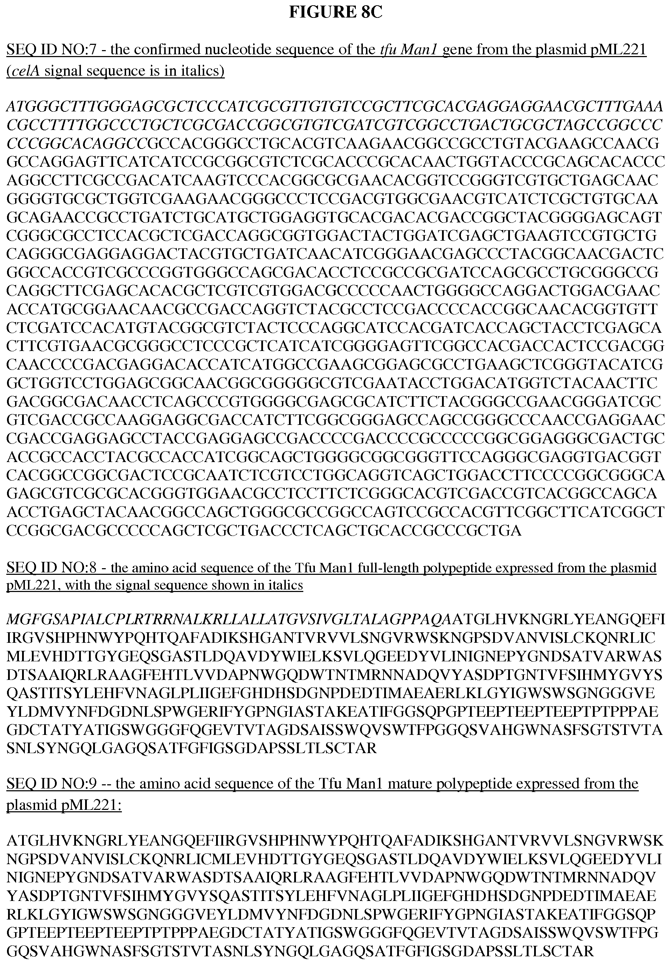

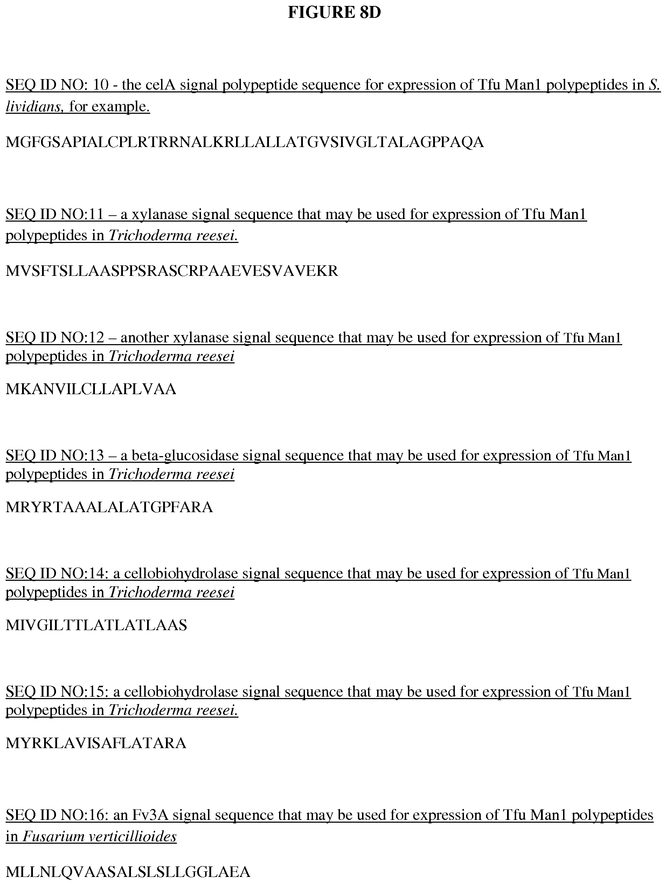

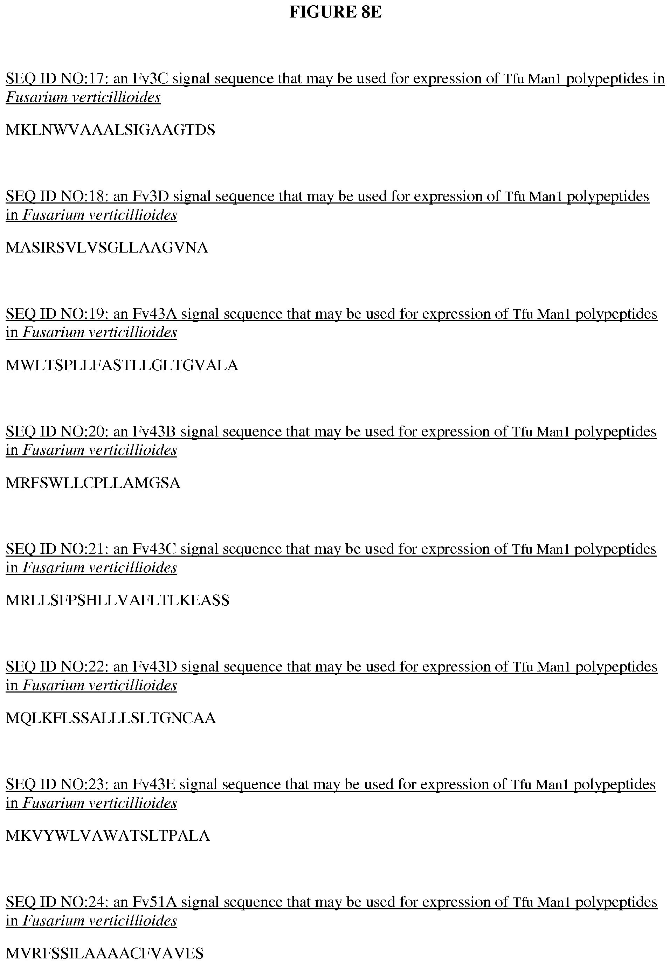

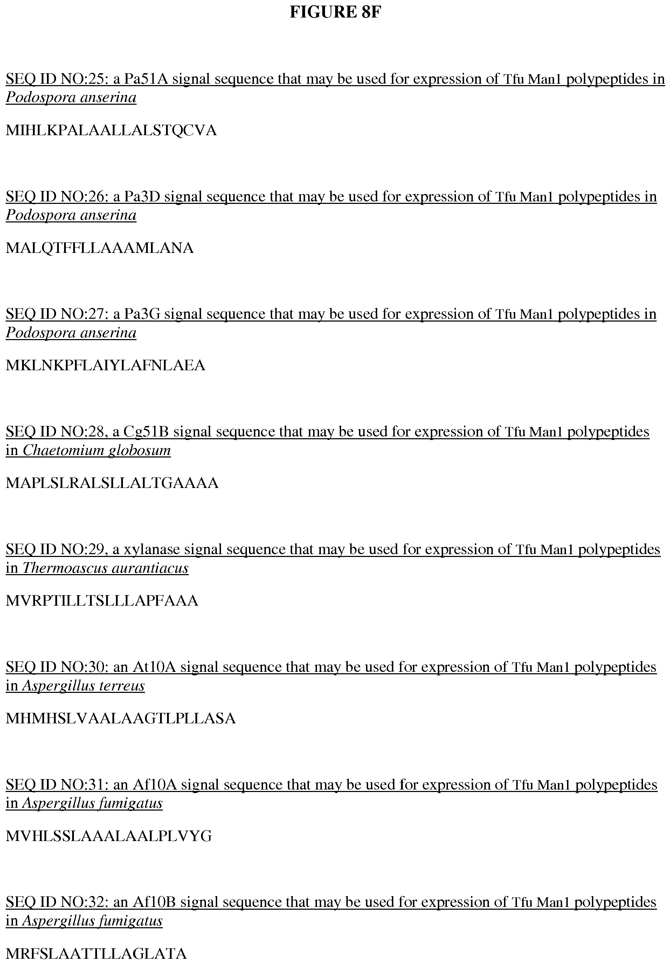

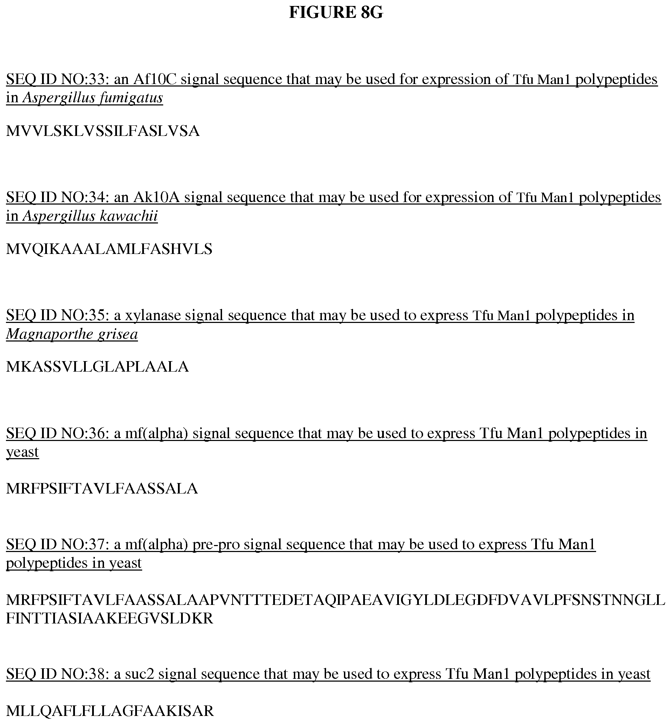

**Figures 8A-8G** depicts the sequences and sequence identifiers of the present disclosure.

### DETAILED DESCRIPTION

### 1. Overview

Described herein are compositions and methods relating to a recombinant beta-mannanase belonging to glycosyl hydrolase family 5 from *Thermobifida fusca.* The present compositions and methods are based, in part, on the observations that recombinant Tfu Man1 polypeptides confer to a cellulase or cellulase and hemicellulase composition comprising at least one cellulase and optionally also at least one other hemicellulase, an improved capacity to hydrolyze a lignocellulosic biomass material or feedstock than other known beta-mannanases of similar pH optimums and/or temperature optimums. The present compositions and methods are also beased on the observation that recombinant Tfu Man1 polypeptides confers rapid viscosity reduction when compositions comprising the polypeptides are used to hydrolyze suitable lignocellulosic biomass substrates, especially when such substrates are treated at high solids levels, and when such substrates contain measurable level of galactoglucomannan (GGM) and/or glucomannan (GM). These features of Tfu Man1 polypeptides make them, or variants thereof, suitable for use in numerous processes, including, for example, in the conversion or hydrolysis of a lignocellulosic biomass feedstock.

Before the present compositions and methods are described in greater detail, it is to be understood that the present compositions and methods are not limited to particular embodiments described, as such may, of course, vary. It is also to be understood that the terminology used herein is for the purpose of describing particular embodiments only, and is not intended to be limiting, since the scope of the present compositions and methods will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the present compositions and methods. The upper and lower limits of these smaller ranges may independently be included in the smaller ranges and are also encompassed within the present compositions and methods, subject to any specifically excluded limit in the stated range. Where the stated range includes one or both of the limits, ranges excluding either or both of those included limits are also included in the present compositions and methods.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number. For example, in connection with a numerical value, the term "about" refers to a range of - 10% to +10% of the numerical value, unless the term is otherwise specifically defined in context. In another example, the phrase a "pH value of about 6" refers to pH values of from 5.4 to 6.6, unless the pH value is specifically defined otherwise.

The headings provided herein are not limitations of the various aspects or embodiments of the present compositions and methods which can be had by reference to the specification as a whole. Accordingly, the terms defined immediately below are more fully defined by reference to the specification as a whole.

The present document is organized into a number of sections for ease of reading; however, the reader will appreciate that statements made in one section may apply to other sections. In this manner, the headings used for different sections of the disclosure should not be construed as limiting.

Unless defined otherwise, all technical and scientific terms used herein have the same meaning as commonly understood by one of ordinary skill in the art to which the present compositions and methods belongs. Although any methods and materials similar or equivalent to those described herein can also be used in the practice or testing of the present compositions and methods, representative illustrative methods and materials are now described.

The citation of any publication is for its disclosure prior to the filing date and should not be construed as an admission that the present compositions and methods are not entitled to antedate such publication by virtue of prior invention. Further, the dates of publication provided may be different from the actual publication dates which may need to be independently confirmed.

In accordance with this detailed description, the following abbreviations and definitions apply. Note that the singular forms "a," "an," and "the" include plural referents unless the context clearly dictates otherwise. Thus, for example, reference to "an enzyme" includes a plurality of such enzymes, and reference to "the dosage" includes reference to one or more dosages and equivalents thereof known to those skilled in the art, and so forth.

It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

The term "recombinant," when used in reference to a subject cell, nucleic acid, polypeptides/enzymes or vector, indicates that the subject has been modified from its native state. Thus, for example, recombinant cells express genes that are not found within the native (non-recombinant) form of the cell, or express native genes at different levels or under different conditions than found in nature. Recombinant nucleic acids may differ from a native sequence by one or more nucleotides and/or are operably linked to heterologous sequences, e.g., a heterologous promoter, signal sequences that allow secretion, etc., in an expression vector. Recombinant polypeptides/enzymes may differ from a native sequence by one or more amino acids and/or are fused with heterologous sequences. A vector comprising a nucleic acid encoding a beta-mannanase is, for example, a recombinant vector.

It is further noted that the term "consisting essentially of," as used herein refers to a composition wherein the component(s) after the term is in the presence of other known component(s) in a total amount that is less than 30% by weight of the total composition and do not contribute to or interferes with the actions or activities of the component(s).

It is further noted that the term "comprising," as used herein, means including, but not limited to, the component(s) after the term "comprising." The component(s) after the term "comprising" are required or mandatory, but the composition comprising the component(s) may further include other non-mandatory or optional component(s).

It is also noted that the term "consisting of," as used herein, means including, and limited to, the component(s) after the term "consisting of." The component(s) after the term "consisting of" are therefore required or mandatory, and no other component(s) are present in the composition.

As will be apparent to those of skill in the art upon reading this disclosure, each of the individual embodiments described and illustrated herein has discrete components and features which may be readily separated from or combined with the features of any of the other several embodiments without departing from the scope or spirit of the present compositions and methods described herein. Any recited method can be carried out in the order of events recited or in any other order which is logically possible.

### 2. Definitions

"Beta-mannanase" means a polypeptide or polypeptide domain of an enzyme that has the ability to catalyze the cleavage or hydrolysis of (1→4)-beta-D-mannosidic linkages of mannans, galactomannans, and glucomannans.

As used herein, "Tfu Man1" or "a Tfu Man1 polypeptide" refers to a beta-mannanase belonging to glycosyl hydrolase family 5 (e.g., a recombinant beta-mannanase) derived from *Thermobifida fusca* (and variants thereof), that confers surprising improvements to a cellulase or cellulase and hemicellulase composition in the composition's capability to hydrolyze a lignocellulosic biomass substrate, optionally pretreated, when compared to other known beta-mannanases of similar pH optimums and/or temperature optimums. The Tfu Man1 polypeptide can substitute a substantial portion, e.g., up to about 20 wt.% (e.g., up to about 20 wt.%, up to about 15 wt.%, up to about 10 wt.%, up to about 9 wt.%, up to about 8 wt.%, up to about 7 wt.%, up to about 6 wt.%, up to about 5 wt.%, up to about 4 wt.%, up to about 3 wt.%, up to about 2 wt.%, up to about 1 wt.%) of a cellulase or cellulase and hemicellulase mixture and achieve equal or better hydrolysis of a given lignocellulosic biomass substrate under the same conditions. This allows the use of less cellulases/hemicellulases and more efficient biomass hydrolysis, thus making the overall cellulosic biomass conversion process more economically feasible and sustainable. The Tfu Man1 polypeptide herein was also surprisingly found to confer rapid viscosity reduction or liquefaction, particularly prominently when the biomass substrate is treated with enzyme at high solids levels. According to aspects of the present compositions and methods, Tfu Man1 polypeptides include those having the amino acid sequence depicted in SEQ ID NO:2, as well as derivative or variant polypeptides having at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to the amino acid sequence of SEQ ID NO:2, or to the mature sequence SEQ ID NO:3 wherein the Tfu Man1 polypeptides not only have beta-mannanase activity and capable of catalyzing the conversion hydrolysis of (1→4)-beta-D-mannosidic linkages of mannans, galactomannans, and glucomannans, but also have higher beta-mannanase activity than other beta-mannases of similar pH optimums and/or temperature optimums, and confer rapid viscosity reduction and liquefaction of high solids biomass substrates, a property that has not been observed with other known beta-mannanases.

"Family 5 glycosyl hydrolase" or "GH5" refers to polypeptides falling within the definition of glycosyl hydrolase family 5 according to the classification by Henrissat, Biochem. J. 280:309-316 (1991), and by Henrissat & Cairoch, Biochem. J., 316:695-696 (1996).

Tfu Man1 polypeptides according to the present compositions and methods described herein can be isolated or purified. By purification or isolation is meant that the Tfu Man1 polypeptide is altered from its natural state by virtue of separating the Tfu Man1 from some or all of the naturally occurring constituents with which it is associated in nature. Such isolation or purification may be accomplished by art-recognized separation techniques such as ion exchange chromatography, affinity chromatography, hydrophobic separation, dialysis, protease treatment, ammonium sulphate precipitation or other protein salt precipitation, centrifugation, size exclusion chromatography, filtration, microfiltration, gel electrophoresis or separation on a gradient to remove whole cells, cell debris, impurities, extraneous proteins, or enzymes undesired in the final composition. It is further possible to then add constituents to the Tfu Man1-containing composition which provide additional benefits, for example, activating agents, anti-inhibition agents, desirable ions, compounds to control pH or other enzymes or chemicals.

As used herein, "microorganism" refers to a bacterium, a fungus, a virus, a protozoan, and other microbes or microscopic organisms.

As used herein, a "derivative" or "variant" of a polypeptide means a polypeptide, which is derived from a precursor polypeptide (e.g., the native polypeptide) by addition of one or more amino acids to either or both the C- and N-terminal end, substitution of one or more amino acids at one or a number of different sites in the amino acid sequence, deletion of one or more amino acids at either or both ends of the polypeptide or at one or more sites in the amino acid sequence, or insertion of one or more amino acids at one or more sites in the amino acid sequence. The preparation of a Tfu Man1 derivative or variant may be achieved in any convenient manner, e.g., by modifying a DNA sequence which encodes the native polypeptides, transformation of that DNA sequence into a suitable host, and expression of the modified DNA sequence to form the derivative/variant Tfu Man1. Derivatives or variants further include Tfu Man1 polypeptides that are chemically modified, e.g., glycosylation or otherwise changing a characteristic of the Tfu Man1 polypeptide. While derivatives and variants of Tfu Man1 are encompassed by the present compositions and methods, such derivates and variants will display improved beta-mannanase activity under the same lignocellulosic biomass substrate hydrolysis conditions, when compared to that of a number of other beta-mannanases having similar pH optimums and/or temperature optimums, for example the ScoMan1 having the sequence of SEQ ID NO:4, or the Bsp Man1, having the sequence of SEQ ID NO:5, or the Msp Man2 of SEQ ID NO:6. In some embodiments, such derivatives and variants will also confer rapid viscosity reduction and liquefaction to a cellulase or cellulase and composition, capable of achieving, for example, at least 10% (e.g., at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 90%, at least 95%, at least 100%, or even more) improved viscosity reduction or higher liquefaction within the same time period after the biomass substrate is subject to an enzyme composition comprising a Tfu Man1 polypeptide herein, as compared to when that same biomass substrate is subject to a counterpart enzyme composition having the same amounts, proportion, and types of enzymes except that the composition does not comprise the Tfu Man1 polypeptide.

In certain aspects, a Tfu Man1 polypeptide of the compositions and methods herein may also encompasses functional fragment of a polypeptide or a polypeptide fragment having beta-mannanase activity, which is derived from a parent polypeptide, which may be the full length polypeptide comprising or consisting of SEQ ID NO:2, or the mature sequence comprising or consisting SEQ ID NO:3. The functional polypeptide may have been truncated either in the N-terminal region, or the C-terminal region, or in both regions to generate a fragment of the parent polypeptide. For the purpose of the present disclosure, a functional fragment must have at least 20%, more preferably at least 30%, 40%, 50%, or preferably, at least 60%, 70%, 80%, or even more preferably at least 90% of the beta-mannanase activity of that of the parent polypeptide.

In certain aspects, a Tfu Man1 derivative/variant will have anywhere from 65% to 99% (or more) amino acid sequence identity to the amino acid sequence of SEQ. ID NO:2, or to the mature sequence SEQ ID NO:3, e.g., 65%, 70%, 75%, 80%, 85%, 86%, 87%, 88%, 89%, 90%, 91%, 92%, 93%, 94%, 95%, 96%, 97%, 98%, or 99% amino acid sequence identity to the amino acid sequence of SEQ. ID NO:2 or to the mature sequence SEQ ID NO:3. In some embodiments, amino acid substitutions are "conservative amino acid substitutions" using L-amino acids, wherein one amino acid is replaced by another biologically similar amino acid. Conservative amino acid substitutions are those that preserve the general charge, hydrophobicity/hydrophilicity, and/or steric bulk of the amino acid being substituted. Examples of conservative substitutions are those between the following groups: Gly/Ala, Val/Ile/Leu, Lys/Arg, Asn/Gln, Glu/Asp, Ser/Cys/Thr, and Phe/Trp/Tyr. A derivative may, for example, differ by as few as 1 to 10 amino acid residues, such as 6-10, as few as 5, as few as 4, 3, 2, or even 1 amino acid residue. In some embodiments, a Tfu Man1 derivative may have an N-terminal and/or C-terminal deletion, where the Tfu Man1 derivative excluding the deleted terminal portion(s) is identical to a contiguous sub-region in SEQ ID NO: 2 or SEQ ID NO:3.

As used herein, "percent (%) sequence identity" with respect to the amino acid or nucleotide sequences identified herein is defined as the percentage of amino acid residues or nucleotides in a candidate sequence that are identical with the amino acid residues or nucleotides in a Tfu Man1 sequence, after aligning the sequences and introducing gaps, if necessary, to achieve the maximum percent sequence identity, and not considering any conservative substitutions as part of the sequence identity.

By "homologue" shall mean an entity having a specified degree of identity with the subject amino acid sequences and the subject nucleotide sequences. A homologous sequence is taken to include an amino acid sequence that is at least 50%, 55%, 60%, 65%, 70%, 75%, 80%, 85%, 86%, 87%, 88%, 89%, 90%, 91%, 92%, 93%, 94%, 95%, 96%, 97%, 98% or even 99% identical to the subject sequence, using conventional sequence alignment tools (*e.g.,* Clustal, BLAST, and the like). Typically, homologues will include the same active site residues as the subject amino acid sequence, unless otherwise specified.

Methods for performing sequence alignment and determining sequence identity are known to the skilled artisan, may be performed without undue experimentation, and calculations of identity values may be obtained with definiteness. See, for example, Ausubel et al., eds. (1995) Current Protocols in Molecular Biology, Chapter 19 (Greene Publishing and Wiley-Interscience, New York); and the ALIGN program (Dayhoff (1978) in Atlas of Protein Sequence and Structure 5:Suppl. 3 (National Biomedical Research Foundation, Washington, D.C.). A number of algorithms are available for aligning sequences and determining sequence identity and include, for example, the homology alignment algorithm of Needleman et al. (1970) J. Mol. Biol. 48:443; the local homology algorithm of Smith et al. (1981) Adv. Appl. Math. 2:482; the search for similarity method of Pearson el al. (1988) Proc. Natal. Acad. Sci. 85:2444; the Smith-Waterman algorithm *(*Meth. Mol. Biol. 70:173-187 (1997); and BLASTP, BLASTN, and BLASTX algorithms (see Altschul et al. (1990) J. Mol. Biol. 215:403-410).

Computerized programs using these algorithms are also available, and include, but are not limited to: ALIGN or Megalign (DNASTAR) software, or WU-BLAST-2 (Altschul et al., (1996) Meth. Enzym., 266:460-480); or GAP, BESTFIT, BLAST, FASTA, and TFASTA, available in the Genetics Computing Group (GCG) package, Version 8, Madison, Wisconsin, USA; and CLUSTAL in the PC/Gene program by Intelligenetics, Mountain View, California. Those skilled in the art can determine appropriate parameters for measuring alignment, including algorithms needed to achieve maximal alignment over the length of the sequences being compared. Preferably, the sequence identity is determined using the default parameters determined by the program. Specifically, sequence identity can determined by using Clustal W (Thompson J.D. et al. (1994) Nucleic Acids Res. 22:4673-4680) with default parameters, i.e.:

| | |
|---|---|
| Gap opening penalty: | 10.0 |
| Gap extension penalty: | 0.05 |
| Protein weight matrix: | BLOSUM series |
| DNA weight matrix: | IUB |
| Delay divergent sequences %: | 40 |
| Gap separation distance: | 8 |
| DNA transitions weight: | 0.50 |
| List hydrophilic residues: | GPSNDQEKR |
| Use negative matrix: | OFF |
| Toggle Residue specific penalties: | ON |
| Toggle hydrophilic penalties: | ON |
| Toggle end gap separation penalty | OFF |

As used herein, "expression vector" means a DNA construct including a DNA sequence which is operably linked to a suitable control sequence capable of affecting the expression of the DNA in a suitable host. Such control sequences may include a promoter to affect transcription, an optional operator sequence to control transcription, a sequence encoding suitable ribosome-binding sites on the mRNA, and sequences which control termination of transcription and translation. Different cell types may be used with different expression vectors. An exemplary promoter for vectors used in *Bacillus subtilis* is the AprE promoter; an exemplary promoter used in *Streptomyces lividans* is the A4 promoter (from *Aspergillus niger);* an exemplary promoter used in *E. coli* is the Lac promoter, an exemplary promoter used in *Saccharomyces cerevisiae* is *PGK1,* an exemplary promoter used in *Aspergillus niger* is *glaA,* and an exemplary promoter for *Trichoderma reesei* is *cbhI.* The vector may be a plasmid, a phage particle, or simply a potential genomic insert. Once transformed into a suitable host, the vector may replicate and function independently of the host genome, or may, under suitable conditions, integrate into the genome itself. In the present specification, plasmid and vector are sometimes used interchangeably. However, the present compositions and methods are intended to include other forms of expression vectors which serve equivalent functions and which are, or become, known in the art. Thus, a wide variety of host/expression vector combinations may be employed in expressing the DNA sequences described herein. Useful expression vectors, for example, may consist of segments of chromosomal, non-chromosomal and synthetic DNA sequences such as various known derivatives of SV40 and known bacterial plasmids, *e.g.,* plasmids from *E. coli* including col E1, pCR1, pBR322, pMb9, pUC 19 and their derivatives, wider host range plasmids, *e.g.,* RP4, phage DNAs *e.g.,* the numerous derivatives of phage λ, *e.g.,* NM989, and other DNA phages, *e.g.,* M13 and filamentous single stranded DNA phages, yeast plasmids such as the 2µ plasmid or derivatives thereof, vectors useful in eukaryotic cells, such as vectors useful in animal cells and vectors derived from combinations of plasmids and phage DNAs, such as plasmids which have been modified to employ phage DNA or other expression control sequences. Expression techniques using the expression vectors of the present compositions and methods are known in the art and are described generally in, for example, Sambrook et al., Molecular Cloning: A Laboratory Manual, Second Edition, Cold Spring Harbor Press (1989). Often, such expression vectors including the DNA sequences described herein are transformed into a unicellular host by direct insertion into the genome of a particular species through an integration event *(see e.g.,* Bennett & Lasure, More Gene Manipulations in Fungi, Academic Press, San Diego, pp. 70-76 (1991) and articles cited therein describing targeted genomic insertion in fungal hosts).

As used herein, "host strain" or "host cell" means a suitable host for an expression vector including DNA according to the present compositions and methods. Host cells useful in the present compositions and methods are generally prokaryotic or eukaryotic hosts, including any transformable microorganism in which expression can be achieved. Specifically, host strains may be *Bacillus subtilis, Bacillus licheniformis, Streptomyces lividans, Escherichia coli, Trichoderma reesei, Saccharomyces cerevisiae, Aspergillus niger, Aspergillus oryzae, Chrysosporium lucknowence, Myceliophthora thermophila,* and various other microbial cells. Host cells are transformed or transfected with vectors constructed using recombinant DNA techniques. Such transformed host cells may be capable of one or both of replicating the vectors encoding Tfu Man1 (and its derivatives or variants (mutants)) and expressing the desired peptide product. In certain embodiments according to the present compositions and methods, "host cell" means both the cells and protoplasts created from the cells of *Trichoderma sp.*

The terms "transformed," "stably transformed," and "transgenic," used with reference to a cell means that the cell contains a non-native *(e.g.,* heterologous) nucleic acid sequence integrated into its genome or carried as an episome that is maintained through multiple generations.

The term "introduced" in the context of inserting a nucleic acid sequence into a cell, means "transfection", "transformation" or "transduction," as known in the art.

A "host strain" or "host cell" is an organism into which an expression vector, phage, virus, or other DNA construct, including a polynucleotide encoding a polypeptide of interest (*e.g.,* a beta-mannanase) has been introduced. Exemplary host strains are microbial cells (*e.g.,* bacteria, filamentous fungi, and yeast) capable of expressing the polypeptide of interest. The term "host cell" includes protoplasts created from cells.

The term "heterologous" with reference to a polynucleotide or polypeptide refers to a polynucleotide or polypeptide that does not naturally occur in a host cell.

The term "endogenous" with reference to a polynucleotide or polypeptide refers to a polynucleotide or polypeptide that occurs naturally in the host cell.

The term "expression" refers to the process by which a polypeptide is produced based on a nucleic acid sequence. The process includes both transcription and translation.

As used herein, "signal sequence" means a sequence of amino acids bound to the N-terminal portion of a protein which facilitates the secretion of the mature form of the protein outside of the cell. This definition of a signal sequence is a functional one. The mature form of the extracellular protein lacks the signal sequence which is cleaved off during the secretion process. While the native signal sequence of Tfu Man1 may be employed in aspects of the present compositions and methods, other non-native signal sequences may be employed (e.g., one selected from SEQ ID NOs:10-38).

The beta-mannanase polypeptides of the invention may be referred to as "precursor," "immature," or "full-length," in which case they include a signal sequence, or may be referred to as "mature," in which case they lack a signal sequence. Mature forms of the polypeptides are generally the most useful. Unless otherwise noted, the amino acid residue numbering used herein refers to the mature forms of the respective beta-mannanase polypeptides. The beta-mannanase polypeptides of the invention may also be truncated to remove the N or C-termini, so long as the resulting polypeptides retain beta-mannanase activity.

The beta-mannanase polypeptides of the invention may also be a "chimeric" or "hybrid" polypeptide, in that it includes at least a portion of a first beta-mannanase polypeptide, and at least a portion of a second beta-mannanase polypeptide (such chimeric beta-mannanase polypeptides may, for example, be derived from the first and second beta-mannanase using known technologies involving the swapping of domains on each of the beta-mannanase). The present beta-mannanase polypeptides may further include heterologous signal sequence, an epitope to allow tracking or purification, or the like. When the term of "heterologous" is used to refer to a signal sequence used to express a polypeptide of interest, it is meant that the signal sequence is, for example, derived from a different microorganism as the polypeptide of interest. Examples of suitable heterologous signal sequences for expressing the Tfu Man1 polypeptides herein, may be, for example, those from *Trichoderma reesei,* other *Trichoderma spp., Aspergillus niger, Aspergillus oryzae,* other *Aspergillus* spp., *Chrysosporium,* and other organisms, those from *Bacillus subtilis, Bacillus licheniformis,* other *Bacillus* species, *E.coli., S. lividians,* or other suitable microbes.

As used herein, "functionally attached" or "operably linked" means that a regulatory region or functional domain having a known or desired activity, such as a promoter, terminator, signal sequence or enhancer region, is attached to or linked to a target (e.g., a gene or polypeptide) in such a manner as to allow the regulatory region or functional domain to control the expression, secretion or function of that target according to its known or desired activity.

As used herein, the terms "polypeptide" and "enzyme" are used interchangeably to refer to polymers of any length comprising amino acid residues linked by peptide bonds. The conventional one-letter or three-letter codes for amino acid residues are used herein. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art.

As used herein, "wild-type" and "native" genes, enzymes, or strains, are those found in nature.

The terms "wild-type," "parental," or "reference," with respect to a polypeptide, refer to a naturally-occurring polypeptide that does not include a man-made substitution, insertion, or deletion at one or more amino acid positions. Similarly, the term "wild-type," "parental," or "reference," with respect to a polynucleotide, refers to a naturally-occurring polynucleotide that does not include a man-made nucleoside change. However, a polynucleotide encoding a wild-type, parental, or reference polypeptide is not limited to a naturally-occurring polynucleotide, but rather encompasses any polynucleotide encoding the wild-type, parental, or reference polypeptide.

The term "derived from" encompasses the terms "originated from," "obtained from," "obtainable from," "isolated from," and "created from," and generally indicates that one specified material find its origin in another specified material or has features that can be described with reference to the another specified material.

As used herein, the term "hybridization conditions" refers to the conditions under which hybridization reactions are conducted. These conditions are typically classified by degree of "stringency" of the conditions under which hybridization is measured. The degree of stringency can be based, for example, on the melting temperature (Tm) of the nucleic acid binding complex or probe. For example, "maximum stringency" typically occurs at about Tm -5°C (5°C below the Tm of the probe); "high stringency" at about 5-10°C below the Tm; "intermediate stringency" at about 10-20°C below the Tm of the probe; and "low stringency" at about 20-25°C below the Tm. Alternatively, or in addition, hybridization conditions can be based upon the salt or ionic strength conditions of hybridization, and/or upon one or more stringency washes, *e.g.:* 6X SSC = very low stringency; 3X SSC = low to medium stringency; 1X SSC = medium stringency; and 0.5X SSC = high stringency. Functionally, maximum stringency conditions may be used to identify nucleic acid sequences having strict identity or near-strict identity with the hybridization probe; while high stringency conditions are used to identify nucleic acid sequences having about 80% or more sequence identity with the probe. For applications requiring high selectivity, it is typically desirable to use relatively stringent conditions to form the hybrids (*e*.*g*., relatively low salt and/or high temperature conditions are used).

As used herein, the term "hybridization" refers to the process by which a strand of nucleic acid joins with a complementary strand through base pairing, as known in the art. More specifically, "hybridization" refers to the process by which one strand of nucleic acid forms a duplex with, *i.e.,* base pairs with, a complementary strand, as occurs during blot hybridization techniques and PCR techniques. A nucleic acid sequence is considered to be "selectively hybridizable" to a reference nucleic acid sequence if the two sequences specifically hybridize to one another under moderate to high stringency hybridization and wash conditions. Hybridization conditions are based on the melting temperature (Tm) of the nucleic acid binding complex or probe. For example, "maximum stringency" typically occurs at about Tm-5°C (5° below the Tm of the probe); "high stringency" at about 5-10°C below the Tm; "intermediate stringency" at about 10-20°C below the Tm of the probe; and "low stringency" at about 20-25°C below the Tm. Functionally, maximum stringency conditions may be used to identify sequences having strict identity or near-strict identity with the hybridization probe; while intermediate or low stringency hybridization can be used to identify or detect polynucleotide sequence homologs.

Intermediate and high stringency hybridization conditions are well known in the art. For example, intermediate stringency hybridizations may be carried out with an overnight incubation at 37°C in a solution comprising 20% formamide, 5 x SSC (150mM NaCl, 15 mM trisodium citrate), 50 mM sodium phosphate (pH 7.6), 5 x Denhardt's solution, 10% dextran sulfate and 20 mg/ml denatured sheared salmon sperm DNA, followed by washing the filters in 1x SSC at about 37 - 50°C. High stringency hybridization conditions may be hybridization at 65°C and 0.1X SSC (where 1X SSC = 0.15 M NaCl, 0.015 M Na₃ citrate, pH 7.0). Alternatively, high stringency hybridization conditions can be carried out at about 42°C in 50% formamide, 5X SSC, 5X Denhardt's solution, 0.5% SDS and 100 µg/ml denatured carrier DNA followed by washing two times in 2X SSC and 0.5% SDS at room temperature and two additional times in 0.1X SSC and 0.5% SDS at 42°C. And very high stringent hybridization conditions may be hybridization at 68°C and 0.1X SSC. Those of skill in the art know how to adjust the temperature, ionic strength, etc. as necessary to accommodate factors such as probe length and the like.

A nucleic acid encoding a variant beta-mannase may have a Tₘ reduced by 1°C - 3°C or more compared to a duplex formed between the nucleotide of SEQ ID NO: 1 and its identical complement.

The phrase "substantially similar" or "substantially identical," in the context of at least two nucleic acids or polypeptides, means that a polynucleotide or polypeptide comprises a sequence that has at least about 90%, at least about 91%, at least about 92%, at least about 93%, at least about 94%, at least about 95%, at least about 96%, at least about 97%, at least about 98%, or even at least about 99% identical to a parent or reference sequence, or does not include amino acid substitutions, insertions, deletions, or modifications made only to circumvent the present description without adding functionality.

As used herein, an "expression vector" refers to a DNA construct containing a DNA sequence that encodes a specified polypeptide and is operably linked to a suitable control sequence capable of effecting the expression of the polypeptides in a suitable host. Such control sequences may include a promoter to effect transcription, an optional operator sequence to control such transcription, a sequence encoding suitable mRNA ribosome binding sites and/or sequences that control termination of transcription and translation. The vector may be a plasmid, a phage particle, or a potential genomic insert. Once transformed into a suitable host, the vector may replicate and function independently of the host genome, or may, in some instances, integrate into the host genome.

The term "recombinant," refers to genetic material (*i*.*e*., nucleic acids, the polypeptides they encode, and vectors and cells comprising such polynucleotides) that has been modified to alter its sequence or expression characteristics, such as by mutating the coding sequence to produce an altered polypeptide, fusing the coding sequence to that of another gene, placing a gene under the control of a different promoter, expressing a gene in a heterologous organism, expressing a gene at a decreased or elevated levels, expressing a gene conditionally or constitutively in a manner different from its natural expression profile, and the like. Generally recombinant nucleic acids, polypeptides, and cells based thereon, have been manipulated by man such that they are not identical to related nucleic acids, polypeptides, and cells found in nature.

A "signal sequence" refers to a sequence of amino acids bound to the N-terminal portion of a polypeptide, and which facilitates the secretion of the mature form of the polypeptide from the cell. The mature form of the extracellular polypeptide lacks the signal sequence which is cleaved off during the secretion process.

The term "selective marker" or "selectable marker," refers to a gene capable of expression in a host cell that allows for ease of selection of those hosts containing an introduced nucleic acid or vector. Examples of selectable markers include but are not limited to antimicrobial substances (e.g., hygromycin, bleomycin, or chloramphenicol) and/or genes that confer a metabolic advantage, such as a nutritional advantage, on the host cell.

The term "regulatory element," refers to a genetic element that controls some aspect of the expression of nucleic acid sequences. For example, a promoter is a regulatory element which facilitates the initiation of transcription of an operably linked coding region. Additional regulatory elements include splicing signals, polyadenylation signals and termination signals.

As used herein, "host cells" are generally cells of prokaryotic or eukaryotic hosts that are transformed or transfected with vectors constructed using recombinant DNA techniques known in the art. Transformed host cells are capable of either replicating vectors encoding the polypeptide variants or expressing the desired polypeptide variant. In the case of vectors, which encode the pre- or pro-form of the polypeptide variant, such variants, when expressed, are typically secreted from the host cell into the host cell medium.

The term "introduced," in the context of inserting a nucleic acid sequence into a cell, means transformation, transduction, or transfection. Means of transformation include protoplast transformation, calcium chloride precipitation, electroporation, naked DNA, and the like as known in the art. (*See,* Chang and Cohen (1979) Mol. Gen. Genet. 168:111-115; Smith et al., (1986) Appl. Env. Microbiol. 51:634; and the review article by Ferrari et al., in Harwood, Bacillus, Plenum Publishing Corporation, pp. 57-72, 1989).

"Fused" polypeptide sequences are connected, *i*.*e*. operably linked, via a peptide bond between two subject polypeptide sequences.

The term "filamentous fungi" refers to all filamentous forms of the subdivision Eumycotina, particulary Pezizomycotina species.

Other technical and scientific terms have the same meaning as commonly understood by one of ordinary skill in the art to which this disclosure pertains *(See, e.g.,* Singleton and Sainsbury, Dictionary of Microbiology and Molecular Biology, 2d Ed., John Wiley and Sons, NY 1994; and Hale and Marham, The Harper Collins Dictionary of Biology, Harper Perennial, NY 1991).

The beta-mannanase enzyme from *Thermobifida fusca* (SEQ ID NO:2) has the following amino acid sequence:

MRKRLAVAAATVLALLASVFALTQPANAATGLHVKNGRLYEANGQEFIIRGVSHPHNW
YPQHTQAFADIKSHGANTVRVVLSNGVRWSKNGPSDVANVISLCKQNRLICMLEVH
DTTGYGEQSGASTLDQAVDYWIELKSVLQGEEDYVLINIGNEPYGNDSATVARWAS
DTSAAIQRLRAAGFEHTLVVDAPNWGQDWTNTMRNNADQVYASDPTGNTVFSIHM
YGVYSQASTITSYLEHFVNAGLPLIIGEFGHDHSDGNPDEDTIMAEAERLKLGYIGWS
WSGNGGGVEYLDMVYNFDGDNLSPWGERIFYGPNGIASTAKEATIFGGSQPGPTEEP
TEEPTEEPTPTPPPAEGDCTATYATIGSWGGGFQGEVTVTAGDSAISSWQVSWTFPGG
QSVAHGWNASFSGTSTVTASNLSYNGQLGAGQSATFGFIGSGDAPSSLTLSCTAR

The mature beta-mannanase enzyme, as based on the removal of the predicted signal peptide sequence if SEQ ID NO:3:

ATGLHVKNGRLYEANGQEFIIRGVSHPHNWYPQHTQAFADIKSHGANTVRVVLSNG
VRWSKNGPSDVANVISLCKQNRLICMLEVHDTTGYGEQSGASTLDQAVDYWIELKS
VLQGEEDYVLINIGNEPYGNDSATVARWASDTSAAIQRLRAAGFEHTLVVDAPNWG
QDWTNTMRNNADQVYASDPTGNTVFSIHMYGVYSQASTITSYLEHFVNAGLPLIIGE
FGHDHSDGNPDEDTIMAEAERLKLGYIGWSWSGNGGGVEYLDMVYNFDGDNLSPW
GERIFYGPNGIASTAKEATIFGGSQPGPTEEPTEEPTEEPTPTPPPAEGDCTATYATIGS
WGGGFQGEVTVTAGDSAISSWQVSWTFPGGQSVAHGWNASFSGTSTVTASNLSYN
GQLGAGQSATFGFIGSGDAPSSLTLSCTAR

A number of other bacterial beta-mannanases having similar pH optimums and/or temperature optimums have been used as benchmark molecules herein, including a beta-mannanase of GH5, called "ScoMan1" herein from *Streptomyces coelicolor* strain A2, having the following amino acid sequence (SEQ ID NO: 4):

MRKPRSTLITTAGMAFAAVLGLLFALAGPSAGRAEAAAGGIHVSNGRVLEGNGSVF
VMRGVNHAYTWYPDRTGSIADIAAKGANTVRVVLSSGGRWTKTSASEVSALIGQCK
ANKVICVLEVHDTTGYGEDGAATSLDQAADYWVSVKSALEGQEDYVVVNIGNEPF
GNTNYTAWTDATKSAIGKLRGAGLDHALMVDAPNWGQDWSGTMRSNAASVFASD
PDRNTVFSVHMYGVYDTAAEVRDYLNAFVGSGLPIVVGEFGDQHSDGNPDEDAIM
ATAQSLGVGYLGWSWSGNGGGVEYLDMVNGFDPNSLTSWGNRIFYGSNGIAATSR
TATVYGGGGGSTGGTAPNGYPYCVNGGASDPDGDGWGWENSRSCVVRGSAADH

Benchmark beta-mannanases also include a beta-mannanase called "Bsp Man1" of GH5 from *Bacillus caldovelox,* having the following amino acid sequence (SEQ ID NO:5)

MNKKWSYTFIALLVSIVCAVVPIFFSQNNVHAKTKREPATPTKDNEFVYRKGDKLMI
GNKEFRFVGTNNYYLHYKSNQMIDDVIESAKKMGIKVIRLWGFFDGMTSENQAHNT
YMQYEMGKYMGEGPIPKELEGAQNGFERLDYTIYKAKQEGIRLVIVLTNNWNNFGG
MMQYVNWIGETNHDLFYTDERIKTAYKNYVHYLINRKNQYTGIIYKNEPTIMAWEL
ANEPRNDSDPTGDTLVRWADEMSTYIKSIDPHHLVAVGDEGFFRRSSGGFNGEGSY
MYTGYNGVDWDRLIALKNIDYGTFHLYPEHWGISPENVEKWGEQYILDHLAAGKK
AKKPVVLEEYGISATGVQNREMIYDTWNRTMFEHGGTGAMFWLLTGIDDNPESADE
NGYYPDYDGFRIVNDHSSVTNLLKTYAKLFNGDRHVEKEPKVYFAFPAKPQDVRGT
YRVKVKVASDQHKVQKVQLQLSSHDEAYTMKYNASFDYYEFDWDTTKEIEDSTVT
LKATATLTNKQTIASDEVTVNIQNASAYEIIKQFSFDSDMNNVYADGTWQANFGIPAI
STPKTRCLRVNVDLPGNADWEEVKVKISPISELSETSRISFDLLLPRVDVNGALRPYIA
LNPGWIKIGVDQYHVNVNDLTTVTIHNQQYKLLHVNVEFNAMPNVNELFLNIVGNK
LAYKGPIYIDNVTLFKKI

Benchmark beta-mannanase further include a beta-mannanase called "Msp Man2" from *Micromonospora sp.,* strain L5, having the following amino acid sequence (SEQ ID NO:6):

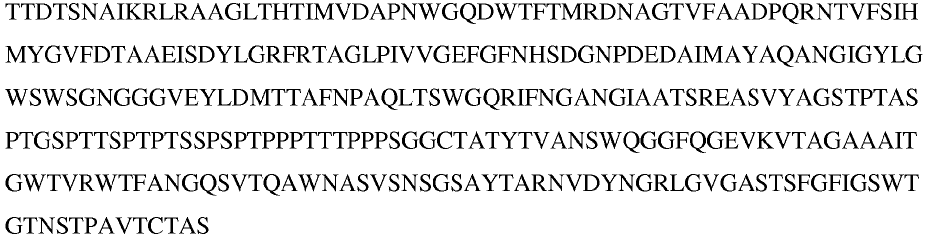

MKKLLSVAGAALLTALAAVFALGQPAHAATGFSVSNGRLYDANGVEFVMRGVNH
AHTWYPQQTSSFANIKALGANTVRVVLSSGDRWTKNSAADVANVISLCKANRMICV
LEVHDTTGYGEDGAATTLAKATDYWLSIADVLKGQEKYVIVNIGNEPFGNQGYSAW

### 3. Beta-Mannanase Polypeptides, Polynucleotides, Vectors, and Host Cells

### A. Tfu Man1 Polypeptides

The present compositions and methods utilise a recombinant Tfu Man1 beta-mannanase polypeptide. An example of a recombinant beta-mannanase polypeptide was isolated from *Thermobifida fusca.* The mature Tfu Man1 polypeptide has the amino acid sequence set forth as SEQ ID NO:3. Similar, substantially similar Tfu Man1 polypeptides may occur in nature, *e.g.,* in other strains or isolates of *Thermobifida fusca,* or *Thermobifida sp.* These and other recombinant Tfu Man1 polypeptides are encompassed by the present compositions and methods.

The recombinant Tfu Man1 polypeptide has at least 65%, 70%, 75%, 80%, 85%, 90%, 91%, 92%, 93%, 94%, 95%, 96%, 97%, 98%, or even at least 99% sequence identity to the amino acid sequence of SEQ ID NO:2 or to the mature sequence SEQ ID NO:3. Sequence identity can be determined by amino acid sequence alignment, e.g., using a program such as BLAST, ALIGN, or CLUSTAL, as described herein.

In certain embodiments, the recombinant Tfu Man1 polypeptides are produced recombinantly, in a microorganism, for example, in a bacterial or fungal host organism, while in others the Tfu Man1 polypeptides are produced synthetically, or are purified from a native source *(e.g., Thermobifida fusca).*

In certain embodiments, the recombinant Tfu Man1 polypeptide includes substitutions that do not substantially affect the structure and/or function of the polypeptide. Examples of these substitutions are conservative mutations, as summarized in Table I.

**Table I. Amino Acid Substitutions**

| **Original Residue** | **Code** | **Acceptable Substitutions** |
|---|---|---|
| Alanine | A | D-Ala, Gly, beta-Ala, L-Cys, D-Cys |
| Arginine | R | D-Arg, Lys, D-Lys, homo-Arg, D-homo-Arg, Met, Ile, D-Met, D-Ile, Orn, D-Orn |
| Asparagine | N | D-Asn, Asp, D-Asp, Glu, D-Glu, Gln, D-Gln |
| Aspartic Acid | D | D-Asp, D-Asn, Asn, Glu, D-Glu, Gln, D-Gln |
| Cysteine | C | D-Cys, S-Me-Cys, Met, D-Met, Thr, D-Thr |
| Glutamine | Q | D-Gln, Asn, D-Asn, Glu, D-Glu, Asp, D-Asp |
| Glutamic Acid | E | D-Glu, D-Asp, Asp, Asn, D-Asn, Gln, D-Gln |
| Glycine | G | Ala, D-Ala, Pro, D-Pro, beta-Ala, Acp |
| Isoleucine | I | D-Ile, Val, D-Val, Leu, D-Leu, Met, D-Met |
| Leucine | L | D-Leu, Val, D-Val, Leu, D-Leu, Met, D-Met |
| Lysine | K | D-Lys, Arg, D-Arg, homo-Arg, D-homo-Arg, Met, D-Met, Ile, D-Ile, Orn, D-Orn |
| Methionine | M | D-Met, S-Me-Cys, Ile, D-Ile, Leu, D-Leu, Val, D-Val |
| Phenylalanine | F | D-Phe, Tyr, D-Thr, L-Dopa, His, D-His, Trp, D-Trp, Trans-3,4, or 5-phenylproline, cis-3,4, or 5-phenylproline |
| Proline | P | D-Pro, L-I-thioazolidine-4- carboxylic acid, D-or L-1-oxazolidine-4-carboxylic acid |
| Serine | S | D-Ser, Thr, D-Thr, allo-Thr, Met, D-Met, Met(O), D-Met(O), L-Cys, D-Cys |
| Threonine | T | D-Thr, Ser, D-Ser, allo-Thr, Met, D-Met, Met(O), D-Met(O), Val, D-Val |
| Tyrosine | Y | D-Tyr, Phe, D-Phe, L-Dopa, His, D-His |
| Valine | V | D-Val, Leu, D-Leu, Ile, D-Ile, Met, D-Met |

Substitutions involving naturally occurring amino acids are generally made by mutating a nucleic acid encoding a recombinant Tfu Man1 polypeptide, and then expressing the variant polypeptide in an organism. Substitutions involving non-naturally occurring amino acids or chemical modifications to amino acids are generally made by chemically modifying a Tfu Man1 polypeptide after it has been synthesized by an organism.

In some embodiments, variant recombinant Tfu Man1 polypeptides are substantially identical to SEQ ID NO:2 or SEQ ID NO:3, meaning that they do not include amino acid substitutions, insertions, or deletions that do not significantly affect the structure, function, or expression of the polypeptide. Such variant recombinant Tfu Man1 polypeptides will include those designed to circumvent the present description. In some embodiments, variants recombinant Tfu Man1 polypeptides, compositions and methods comprising these variants are not substantially identical to SEQ ID NO:2 or SEQ ID NO:3, but rather include amino acid substitutions, insertions, or deletions that affect, in certain circumstances, substantially, the structure, function, or expression of the polypeptide herein such that improved characteristics, including, e.g., improved specific activity to hydrolyze a mannan-containing lignocellulosic substrate, more rapid viscosity reduction when used to treat high solids biomass substrates, improved expression in a desirable host organism, improved thermostability, pH stability, etc, as compared to that of a polypeptide of SEQ ID NO:2 or SEQ ID NO:3 can be achieved.

The recombinant Tfu Man1 polypeptide (including a variant thereof) has beta-mannanase activity. Beta-mannanase activity can be determined using an assay measuring the release of reducing sugars from a galactomannan substrate, for example, in accordance with the description of Example 5. Beta-mannanase activity can be determined by combining with a cellulase or cellulase and hemicellulase mixture, followed by using such a mixture to treat a suitable mannan-containing biomass substrate, such as, for example, a woody substrate, etc., in accordance with the protocols and conditions described in, for example, Example 9, or by suitable assays, or methods of activity measurement known in the art.

Recombinant Tfu Man1 polypeptides include fragments of "full-length" Tfu Man1 polypeptides that retain beta-mannanase activity. Preferably those functional fragments *(i.e.,* fragments that retain beta-mannanase activity) are at least 80 amino acid residues in length (*e.g.,* at least 80 amino acid residues, at least 100 amino acid residues, at least 120 amino acid residues, at least 140 amino acid residues, at least 160 amino acid residues, at least 180 amino acid residues, at least 200 amino acid residues, at least 220 amino acid residues, at least 240 amino acid residues, at least 260 amino acid residues, at least 280 amino acid residues, at least 300 amino acid residues, at least 350 residues in length or longer). Such fragments suitably retain the active site of the full-length precursor polypeptides or full length mature polypeptides but may have deletions of non-critical amino acid residues. The activity of fragments can be readily determined using the methods of measuring beta-mannanase activity described herein, for example the assay described in Example 5, and the hydrolysis performance measurements as those described in Example 9, or by suitable assays or other means of activity measurements known in the art.

In some embodiments, the Tfu Man1 amino acid sequences and derivatives are produced as an N- and/or C-terminal fusion protein, for example, to aid in extraction, detection and/or purification and/or to add functional properties to the Tfu Man1 polypeptides. Examples of fusion protein partners include, but are not limited to, glutathione-S-transferase (GST), 6XHis, GAL4 (DNA binding and/or transcriptional activation domains), FLAG-, MYC-tags or other tags known to those skilled in the art. In some embodiments, a proteolytic cleavage site is provided between the fusion protein partner and the polypeptide sequence of interest to allow removal of fusion sequences. Suitably, the fusion protein does not hinder the activity of the recombinant Tfu Man1 polypeptide. In some embodiments, the recombinant Tfu Man1 polypeptide is fused to a functional domain including a leader peptide, propeptide, binding domain and/or catalytic domain. Fusion proteins are optionally linked to the recombinant Tfu Man1 polypeptide through a linker sequence that joins the Tfu Man1 polypeptide and the fusion domain without significantly affecting the properties of either component. The linker optionally contributes functionally to the intended application.

The present disclosure provides host cells that are engineered to express one or more Tfu Man1 polypeptides of the disclosure. Suitable host cells include cells of any microorganism *(e.g.,* cells of a bacterium, a protist, an alga, a fungus *(e.g.,* a yeast or filamentous fungus), or other microbe), and are preferably cells of a bacterium, a yeast, or a filamentous fungus.

Suitable host cells of the bacterial genera include, but are not limited to, cells of *Escherichia, Bacillus, Lactobacillus, Pseudomonas,* and *Streptomyces.* Suitable cells of bacterial species include, but are not limited to, cells of *Escherichia coli, Bacillus subtilis, Bacillus licheniformis, Lactobacillus brevis, Pseudomonas aeruginosa,* and *Streptomyces lividans.*

Suitable host cells of the genera of yeast include, but are not limited to, cells of *Saccharomyces, Schizosaccharomyces, Candida, Hansenula, Pichia, Kluyveromyces,* and *Ph*affia. Suitable cells of yeast species include, but are not limited to, cells of *Saccharomyces cerevisiae, Schizosaccharomyces pombe, Candida albicans, Hansenula polymorpha, Pichia pastoris, P. canadensis, Kluyveromyces marxianus,* and *Phaffia rhodozyma.*

Suitable host cells of filamentous fungi include all filamentous forms of the subdivision *Eumycotina.* Suitable cells of filamentous fungal genera include, but are not limited to, cells of *Acremonium, Aspergillus, Aureobasidium, Bjerkandera, Ceriporiopsis, Chrysoporium, Coprinus, Coriolus, Corynascus, Chaertomium, Cryptococcus, Filobasidium, Fusarium, Gibberella, Humicola, Maguaporthe, Mucor, Myceliophthora, Mucor, Neocallimastix, Neurospora, Paecilomyces, Penicillium, Phanerochaete, Phlebia, Piromyces, Pleurotus,Scytaldium, Schizophyllum, Sporotrichum, Talaromyces, Thermoascus, Thielavia, Tolypocladium, Trametes,* and *Trichoderma.*

Suitable cells of filamentous fungal species include, but are not limited to, cells of *Aspergillus awamori, Aspergillus fumigatus, Aspergillus foetidus, Aspergillus japonicus, Aspergillus nidulans, Aspergillus niger, Aspergillus oryzae, Chrysosporium lucknowense, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Bjerkandera adusta, Ceriporiopsis aneirina, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Coprinus cinereus, Coriolus hirsutus, Humicola insolens, Humicola lanuginosa, Mucor miehei, Myceliophthora thermophila, Neurospora crassa, Neurospora intermedia, Penicillium purpurogenum, Penicillium canescens, Penicillium solitum, Penicillium funiculosum Phanerochaete chrysosporium, Phlebia radiate, Pleurotus eryngii, Talaromyces flavus, Thielavia terrestris, Trametes villosa, Trametes versicolor, Trichoderma harzianum, Trichoderma koningii, Trichoderma longibrachiatum, Trichoderma reesei,* and *Trichoderma viride.*

Methods of transforming nucleic acids into these organisms are known in the art. For example, a suitable procedure for transforming *Aspergillus* host cells is described in EP 238 023.

In some embodiments, the recombinant Tfu Man1 polypeptide is fused to a signal peptide to, for example, facilitate extracellular secretion of the recombinant Tfu Man1 polypeptide. For example, in certain embodiments, the signal peptide is a non-native signal peptide such as the *S. lividian* celA signal peptide of SEQ ID NO:10. In particular embodiments, the recombinant Tfu Man1 polypeptide is expressed in a heterologous organism as a secreted polypeptide. The compositions and methods herein thus encompass methods for expressing a Tfu Man1 polypeptide as a secreted polypeptide in a heterologous organism.

The disclosure also provides expression cassettes and/or vectors comprising the above-described nucleic acids. Suitably, the nucleic acid encoding a Tfu Man1 polypeptide of the disclosure is operably linked to a promoter. Promoters are well known in the art. Any promoter that functions in the host cell can be used for expression of a beta-mannanase and/or any of the other nucleic acids of the present disclosure. Initiation control regions or promoters, which are useful to drive expression of a beta-mannanase nucleic acids and/or any of the other nucleic acids of the present disclosure in various host cells are numerous and familiar to those skilled in the art *(see,* for example, WO 2004/033646 and references cited therein). Virtually any promoter capable of driving these nucleic acids can be used.

Specifically, where recombinant expression in a filamentous fungal host is desired, the promoter can be a filamentous fungal promoter. The nucleic acids can be, for example, under the control of heterologous promoters. The nucleic acids can also be expressed under the control of constitutive or inducible promoters. Examples of promoters that can be used include, but are not limited to, a cellulase promoter, a xylanase promoter, the 1818 promoter (previously identified as a highly expressed protein by EST mapping *Trichoderma*)*.* For example, the promoter can suitably be a cellobiohydrolase, endoglucanase, or beta-glucosidase promoter. A particulary suitable promoter can be, for example, a *T. reesei* cellobiohydrolase, endoglucanase, or beta-glucosidase promoter. For example, the promoter is a cellobiohydrolase I (*cbh*1) promoter. Non-limiting examples of promoters include a *cbh1, cbh2, egll, egl2, egl3, egl4, egl5, pki1, gpd1, xyn1,* or *xyn2* promoter. Additional non-limiting examples of promoters include a *T. reesei cbh1, cbh2, egl1, egl2, egl3, egl4, egl5, pkil, gpd1, xyn1,* or *xyn2* promoter.

The nucleic acid sequence encoding a Tfu Man1 polypeptide herein can be included in a vector. In some aspects, the vector contains the nucleic acid sequence encoding the Tfu Man1 polypeptide under the control of an expression control sequence. In some aspects, the expression control sequence is a native expression control sequence. In some aspects, the expression control sequence is a non-native expression control sequence. In some aspects, the vector contains a selective marker or selectable marker. In some aspects, the nucleic acid sequence encoding the Tfu Man1 polypeptide is integrated into a chromosome of a host cell without a selectable marker.

Suitable vectors are those which are compatible with the host cell employed. Suitable vectors can be derived, for example, from a bacterium, a virus (such as bacteriophage T7 or a M-13 derived phage), a cosmid, a yeast, or a plant. Suitable vectors can be maintained in low, medium, or high copy number in the host cell. Protocols for obtaining and using such vectors are known to those in the art (s*ee,* for example, Sambrook et al., Molecular Cloning: A Laboratory Manual, 2nd ed., Cold Spring Harbor, 1989).

In some aspects, the expression vector also includes a termination sequence. Termination control regions may also be derived from various genes native to the host cell. In some aspects, the termination sequence and the promoter sequence are derived from the same source.

A nucleic acid sequence encoding a Tfu Man1 polypeptide can be incorporated into a vector, such as an expression vector, using standard techniques (Sambrook et al., Molecular Cloning: A Laboratory Manual, Cold Spring Harbor, 1982).

In some aspects, it may be desirable to over-express a Tfu Man1 polypeptide and/or one or more of any other nucleic acid described in the present disclosure at levels far higher than currently found in naturally-occurring cells. In some embodiments, it may be desirable to under-express *(e.g.,* mutate, inactivate, or delete) an endogenous beta-mannanase and/or one or more of any other nucleic acid described in the present disclosure at levels far below that those currently found in naturally-occurring cells.

### B. Tfu Man1 -Encoding Polynucleotides

Another aspect of the disclosure is a polynucleotide or a nucleic acid sequence that encodes a recombinant Tfu Man1 polypeptide having beta-mannanase activity. In some embodiments the polynucleotide is provided in the context of an expression vector for directing the expression of a Tfu Man1 polypeptide in a heterologous organism, such as one identified herein. The polynucleotide that encodes a recombinant Tfu Man1 polypeptide may be operably-linked to regulatory elements *(e.g.,* a promoter, terminator, enhancer, and the like) to assist in expressing the encoded polypeptides.

An example of a polynucleotide sequence encoding a recombinant Tfu Man1 polypeptide has the nucleotide sequence of SEQ ID NO: 1. Similar, including substantially identical, polynucleotides encoding recombinant Tfu Man1 polypeptides and variants may occur in nature, *e.g.,* in other strains or isolates of *Thermobifida fusca,* or *Thermobifida sp..* In view of the degeneracy of the genetic code, it will be appreciated that polynucleotides having different nucleotide sequences may encode the same Tfu Man1 polypeptides, variants, or fragments.

In some examples disclosed herein the polynucleotide that encodes a recombinant Tfu Man1 polypeptide is fused in frame behind (*i.e.,* downstream of) a coding sequence for a signal peptide for directing the extracellular secretion of a recombinant Tfu Man1 polypeptide. As described herein, the term "heterologous" when used to refer to a signal sequence used to express a polypeptide of interest, it is meant that the signal sequence and the polypeptide of interest are from different organisms. Heterologous signal sequences include, for example, those from other fungal cellulase genes, such as, *e.g.,* the signal sequence of *Trichoderma reesei* CBH1. Expression vectors may be provided in a heterologous host cell suitable for expressing a recombinant Tfu Man1 polypeptide, or suitable for propagating the expression vector prior to introducing it into a suitable host cell.

In some examples disclosed herein, polynucleotides encoding recombinant Tfu Man1 polypeptides hybridize to the polynucleotide of SEQ ID NO: 1 (or to the complement thereof) under specified hybridization conditions. Examples of conditions are intermediate stringency, high stringency and extremely high stringency conditions, which are described herein.

Tfu Man1 polynucleotides may be naturally occurring or synthetic *(i.e.,* man-made), and may be codon-optimized for expression in a different host, mutated to introduce cloning sites, or otherwise altered to add functionality.

The nucleic acid sequence encoding the coding region of Tfu Man1 polypeptide derived from *Thermobifida fusca* is as follows (SEQ ID NO: 1), wherein the nucleic acid sequence encoding the predicted signal peptide sequence is italicized:

ATGAGAAAACGTCTCGCGGTCGCCGCAGCAACCGTCCTTGCGCTGCTGGCGTCCGTC
TTTGCCCTCACGCAGCCGGCGAACGCGGCCACCGGGCTCCACGTCAAGAACGGCC
GCCTGTATGAGGCCAACGGCCAGGAGTTCATCATCCGTGGCGTCAGCCACCCCC
ACAACTGGTACCCCCAGCACACCCAGGCGTTCGCCGACATCAAGTCGCACGGCG
CCAACACCGTCCGGGTGGTGCTGAGCAACGGTGTCCGGTGGAGCAAGAACGGTC
CTTCTGACGTCGCCAACGTCATCTCCCTGTGCAAGCAGAACCGCCTTATCTGCAT
GCTGGAGGTGCACGACACCACCGGCTACGGCGAGCAGAGCGGGGCCTCCACGCT
CGACCAGGCCGTCGACTACTGGATCGAGCTGAAGAGCGTGCTCCAGGGCGAGGA
GGACTATGTCCTCATCAACATCGGCAACGAGCCCTACGGCAACGACTCCGCGAC
CGTCGCCCGCTGGGCGTCGGACACCTCCGCCGCCATCCAGCGGCTGCGCGCCGCC
GGATTCGAGCACACCCTCGTGGTGGACGCCCCCAACTGGGGCCAGGACTGGACG
AACACCATGCGGAACAACGCCGACCAGGTGTACGCCAGCGACCCCACCGGCAAC
ACCGTCTTCTCGATCCACATGTACGGCGTCTACTCCCAGGCGTCCACGATCACCA
GCTACCTGGAGCACTTCGTCAACGCGGGCCTGCCGCTCATCATCGGCGAGTTCGG
CCACGACCACTCCGACGGCAACCCCGACGAGGACACGATCATGGCCGAGGCCGA
GCGGCTCAAGCTGGGCTACATCGGCTGGTCGTGGAGCGGCAACGGCGGCGGGT
CGAGTACCTCGACATGGTGTACAACTTCGACGGCGACAACCTGAGCCCGTGGGG
CGAGCGGATCTTCTACGGCCCTAACGGTATTGCGAGCACGGCCAAGGAGGCCAC
GATCTTCGGCGGCTCCCAGCCGGGCCCGACCGAGGAGCCGACGGAAGAGCCGAC
CGAGGAGCCGACTCCGACTCCGCCTCCCGCGGAGGGCGACTGCACCGCCACCTA
CGCCACCATCGGCAGCTGGGGCGGCGGCTTCCAGGGCGAGGTGACGGTCACCGC
GGGCGACTCCGCGATCTCCTCGTGGCAGGTGAGCTGGACCTTCCCCGGCGGCCA
GAGCGTGGCCCACGGCTGGAACGCCAGCTTCAGCGGCACGAGCACGGTGACGGC
CAGCAACCTGTCCTACAACGGCCAGCTGGGTGCGGACAGTCCGCCACGTTCGG
CTTCATCGGCTCGGGCGACGCGCCTAGCTCGCTGACGCTGAGCTGCACCGCTCGC
TGA

As is well known to those of ordinary skill in the art, due to the degeneracy of the genetic code, polynucleotides having significantly different sequences can nonetheless encode identical, or nearly identical, polypeptides. As such, also disclosed are polynucleotides encoding Tfu Man1 polypeptides that contain a nucleic acid sequence that is at least 65% identical to SEQ ID NO: 1, including at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identical to SEQ ID NO: 1. In some embodiments, Tfu Man1 polypeptides contain a nucleic acid sequence that is identical to SEQ ID NO: 1.

In some examples of the disclosure polynucleotides may include a sequence encoding a signal peptide. Many convenient signal sequences may be suitably employed.

### C. Purification from Natural Isolates

The Tfu Man1 polypeptides can be purified from natural isolates *(e.g.,* from a strain of *Thermobifida fusca*) by known and commonly employed methods. For example, cells containing a Tfu Man1 polypeptide can be disrupted by various physical or chemical means, such as freeze-thaw cycling, sonication, mechanical disruption, or cell lysing agents. Cell supernatants may be collected (for example from cells that secrete the protein into the medium). The Tfu Man1 polypeptide can be recovered from the medium and/or lysate by conventional techniques including separations of the cells/debris from the medium by centrifugation, filtration, and precipitation of the proteins in the supernatant or filtrate with a salt, for example, ammonium sulphate. The Tfu Man1 polypeptide can then be purified from the disrupted cells by procedures such as: fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; and affinity chromatography. Various methods of protein purification may be employed and such methods are known in the art and described for example in Deutscher, Methods in Enzymology, 182 (1990); Scopes, Protein Purification: Principles and Practice, Springer-Verlag, New York (1982).

### D. Chemical Synthesis

Alternatively, the Tfu Man1 polypeptide sequence, or portions thereof, may be produced by direct peptide synthesis using solid-phase techniques *(see, e.g.,* Stewart et al., Solid-Phase Peptide Synthesis, W.H. Freeman Co., San Francisco, CA (1969); Merrifield, J. Am. Chem. Soc., 85:2149-2154 (1963)). *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of Tfu Man1 may be chemically synthesized separately and combined using chemical or enzymatic methods to produce a full-length Tfu Man1.

### E. Recombinant Methods of Making

### Isolation of DNA Encoding the Tfu Man1 polypeptide

DNA encoding a Tfu Man1 polypeptide may be obtained from a cDNA library prepared from a microorganism believed to possess the Tfu Man1 mRNA *(e.g., T. fusca)* and to express it at a detectable level. The Tfu Man1-encoding gene may also be obtained from a genomic library or by oligonucleotide synthesis.

Libraries can be screened with probes (such as antibodies to a Tfu Man1 or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding Tfu Man1 is to use PCR methodology (Sambrook et al., supra; Dieffenbach et al., PCR Primer:A Laboratory Manual (Cold Spring Harbor Laboratory Press, 1995)).

In known techniques for screening a cDNA library, the oligonucleotide sequences selected as probes should be of sufficient length and sufficiently unambiguous that false positives are minimized. The oligonucleotide can be labeled such that it can be detected upon hybridization to DNA in the library being screened. Methods of labeling are well known in the art, and include the use of radiolabels like ³²P-labeled ATP, biotinylation or enzyme labeling. Hybridization conditions, including moderate stringency and high stringency, are provided in Sambrook et al., Molecular Cloning: A Laboratory Manual (New York: Cold Spring Harbor Laboratory Press, 1989).

Nucleic acids having protein coding sequence may be obtained by screening selected cDNA or genomic libraries using the deduced amino acid sequence disclosed herein for the first time, and, if necessary, using conventional primer extension procedures as described in Sambrook et al., Molecular Cloning: A Laboratory Manual (New York: Cold Spring Harbor Laboratory Press, 1989), to detect precursors and processing intermediates of mRNA that may not have been reverse-transcribed into cDNA.

### Selection and Transformation of Host Cells

Host cells are transfected or transformed with expression or cloning vectors described herein for Tfu Man1 production. The host cells are cultured in conventional nutrient media modified as appropriate for inducing promoters, selecting transformants, or amplifying the genes encoding the desired sequences. The culture conditions, such as media, temperature, pH and the like, can be selected by the ordinarily skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook et al., Molecular Cloning: A Laboratory Manual (New York: Cold Spring Harbor Laboratory Press, 1989).

Methods of transfection are known to the ordinarily skilled artisan, for example, CaPO₄ and electroporation. Depending on the host cell used, transformation is performed using standard techniques appropriate to such cells. The calcium treatment employing calcium chloride, as described in Sambrook et al., Molecular Cloning: A Laboratory Manual (New York: Cold Spring Harbor Laboratory Press, 1989), or electroporation is generally used for prokaryotes or other cells that contain substantial cell-wall barriers. Infection with *Agrobacterium tumefaciens* is used for transformation of certain plant cells, as described by Shaw et al., Gene, 23:315 (1983) and WO 89/05859 published 29 June 1989. Transformations into yeast can be carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, microporation, biolistic bombardment, bacterial protoplast fusion with intact cells, or polycations, *e*.*g*., polybrene, polyornithine, may also be used.

Suitable host cells for cloning or expressing the DNA in the vectors herein include prokaryote, yeast, or filamentous fungal cells. Suitable prokaryotes include but are not limited to eubacteria, such as Gram-negative or Gram-positive organisms, for example, Enterobacteriaceae such as *E. coli.* Various *E. coli* strains are publicly available, such as *E*. *coli* K12 strain MM294 (ATCC 31,446); *E. coli* X1776 (ATCC 31,537); *E. coli* strain W3110 (ATCC 27,325) and K5 772 (ATCC 53,635). In addition to prokaryotes, eukaryotic microorganisms such as filamentous fungi or yeast are suitable cloning or expression hosts for vectors encoding Tfu Man1 polypeptides. *Saccharomyces cerevisiae* is a commonly used lower eukaryotic host microorganism.

In some examples of the disclosure the microorganism to be transformed includes a strain derived from *Trichoderma* spp. or *Aspergillus* spp. Exemplary strains include *T. reesei* which is useful for obtaining overexpressed protein or *Aspergillus niger* var. *awamori.* For example, *Trichoderma* strain RL-P37, described by Sheir-Neiss et al. in Appl. Microbiol. Biotechnology, 20 (1984) pp. 46-53 is known to secrete elevated amounts of cellulase enzymes. Functional equivalents of RL-P37 include *Trichoderma reesei (longibrachiatum)* strain RUT-C30 (ATCC No. 56765) and strain QM9414 (ATCC No. 26921). Another example includes overproducing mutants as described in Ward et al. in Appl. Microbiol. Biotechnology 39:738-743 (1993). For example, it is contemplated that these strains would also be useful in overexpressing a *Thermobifida fusca* Tfu Man1 polypeptide, or a variant thereof. The selection of the appropriate host cell is deemed to be within the skill in the art.

### Preparation and Use of a Replicable Vector

DNA encoding the Tfu Man1 protein or derivatives thereof (as described above) is prepared for insertion into an appropriate microorganism. According to the present compositions and methods, DNA encoding a Tfu Man1 polypeptide includes all of the DNA necessary to encode for a protein which has functional Tfu Man1 activity. As such, embodiments of the present compositions and methods include DNA encoding a Tfu Man1 polypeptide derived from *Thermobifida sp.,* including, *Thermobifida fusca.*

The DNA encoding Tfu Man1 may be prepared by the construction of an expression vector carrying the DNA encoding Tfu Man1. The expression vector carrying the inserted DNA fragment encoding the Tfu Man1 may be any vector which is capable of replicating autonomously in a given host organism or of integrating into the DNA of the host, typically a plasmid, cosmid, viral particle, or phage. Various vectors are publicly available. It is also contemplated that more than one copy of DNA encoding a Tfu Man1 may be recombined into the strain to facilitate overexpression.

In certain examples of the disclosure DNA sequences for expressing Tfu Man1 include the promoter, gene coding region, and terminator sequence all originate from the native gene to be expressed. Gene truncation may be obtained by deleting away undesired DNA sequences (e.g., coding for unwanted domains) to leave the domain to be expressed under control of its native transcriptional and translational regulatory sequences. A selectable marker can also be present on the vector allowing the selection for integration into the host of multiple copies of the Tfu Man1 gene sequences.

In other examples of the disclosure the expression vector is preassembled and contains sequences required for high level transcription and, in some cases, a selectable marker. It is contemplated that the coding region for a gene or part thereof can be inserted into this general purpose expression vector such that it is under the transcriptional control of the expression cassette's promoter and terminator sequences. For example, pTEX is such a general purpose expression vector. Genes or part thereof can be inserted downstream of the strong *cbh*1 promoter.

In the vector, the DNA sequence encoding the Tfu Man1 of the present compositions and methods should be operably linked to transcriptional and translational sequences, *e.g.,* a suitable promoter sequence and signal sequence in reading frame to the structural gene. The promoter may be any DNA sequence which shows transcriptional activity in the host cell and may be derived from genes encoding proteins either homologous or heterologous to the host cell. The signal peptide provides for extracellular production (secretion) of the Tfu Man1 or derivatives thereof. The DNA encoding the signal sequence can be that which is naturally associated with the gene to be expressed. However the signal sequence from any suitable source, for example an exo-cellobiohydrolases or endoglucanase from *Trichoderma,* a xylanase from a bacterial species, *e.g.,* from *Streptomyces coelicolor,* etc., are contemplated in the present compositions and methods.

The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

A desired Tfu Man 1 polypeptide may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector or it may be a part of the Tfu Man1-encoding DNA that is inserted into the vector. The signal sequence may be a prokaryotic signal sequence selected, for example, from the group of the alkaline phosphatase, penicillinase, lpp, or heat-stable enterotoxin II leaders. For yeast secretion the signal sequence may be, *e.g.,* the yeast invertase leader, alpha factor leader (including *Saccharomyces* and *Kluyveromyces* α-factor leaders, the latter described in U.S. Patent No. 5,010,182), or acid phosphatase leader, the *C*. *albicans* glucoamylase leader (EP 362,179 published 4 April 1990), or the signal described in WO 90/13646 published 15 November 1990.

Both expression and cloning vectors may contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria and the 2µ plasmid origin is suitable for yeast.

Expression and cloning vectors will typically contain a selection gene, also termed a selectable marker. Typical selection genes encode proteins that (a) confer resistance to antibiotics or other toxins, *e.g.,* ampicillin, neomycin, methotrexate, or tetracycline, (b) complement auxotrophic deficiencies, or (c) supply critical nutrients not available from complex media, *e.g.,* the gene encoding D-alanine racemase for *Bacilli.* A suitable selection gene for use in yeast is the *trp*1 gene present in the yeast plasmid YRp7 (Stinchcomb et al., Nature, 282:39 (1979); Kingsman et al., Gene, 7:141 (1979); Tschemper et al., Gene, 10:157 (1980)). The *trp* 1 gene provides a selection marker for a mutant strain of yeast lacking the ability to grow in tryptophan, for example, ATCC No. 44076 or PEP4-1 (Jones, Genetics, 85:12 (1977)). An exemplary selection gene for use in *Trichoderma sp* is the *pyr4* gene.

Expression and cloning vectors usually contain a promoter operably linked to the Tfu Man1-encoding nucleic acid sequence. The promoter directs mRNA synthesis. Promoters recognized by a variety of potential host cells are well known. Promoters include a fungal promoter sequence, for example, the promoter of the *cbh1* or *egl1* gene.

Promoters suitable for use with prokaryotic hosts include the β-lactamase and lactose promoter systems (Chang et al., Nature, 275:615 (1978); Goeddel et al., Nature, 281:544 (1979)), alkaline phosphatase, a tryptophan (trp) promoter system (Goeddel, Nucleic Acids Res., 8:4057 (1980); EP 36,776), and hybrid promoters such as the tac promoter (deBoer et al., Proc. Natl. Acad. Sci. USA, 80:21-25 (1983)). Additional promoters, *e.g.,* the A4 promoter from A. *niger,* also find use in bacterial expression systems, e.g., in S. *lividans.* Promoters for use in bacterial systems also may contain a Shine-Dalgarno (S.D.) sequence operably linked to the DNA encoding a Tfu Man1 polypeptide.

Examples of suitable promoting sequences for use with yeast hosts include the promoters for 3-phosphoglycerate kinase (Hitzeman et al., J. Biol. Chem., 255:2073 (1980)) or other glycolytic enzymes (Hess et al., J. Adv. Enzyme Reg., 7:149 (1968); Holland, Biochemistry, 17:4900 (1978)), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

Expression vectors used in eukaryotic host cells (e.g. yeast, fungi, insect, plant) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding a Tfu Man1 polypeptide.

### Purification of a Tfu Man1 polypeptide

Forms of Tfu Man1 polypeptides may be recovered from culture medium or from host cell lysates by the methods described above for isolation and purification from natural isolates. Additional techniques can be used depending on the host cell employed and any variant structures in the recombinant enzyme. For example, if the recombinant enzyme is membrane-bound, it can be released from the membrane using a suitable detergent solution *(e.g.* Triton-X 100) or by enzymatic cleavage. Purification of recombinant enzyme may also employ protein A Sepharose columns to remove contaminants such as IgG and metal chelating columns to bind epitope-tagged forms of the Tfu Man1 polypeptide. The purification step(s) selected will depend, for example, on the nature of the production process used, the particular Tfu Man1 polypeptide that is produced, and any variant structure for the recombinant enzyme. Antibodies directed to a Tfu Man1 polypeptide or epitope tags thereon may also be employed to purify the protein, e.g., anti- Tfu Man1 antibodies attached to a solid support.

### 4. Derivatives of Tfu Man1

As described above, in addition to the native sequence of Tfu Man1 described herein (e.g., as depicted in full length as SEQ ID NO:2, and in the mature form as SEQ ID NO: 3), it is contemplated that Tfu Man1 derivatives can be prepared with altered amino acid sequences. In general, Tfu Man1 derivatives would be capable of conferring, as a native Tfu Man1 polypeptide, to a cellulase or cellulase and hemicellulase mixture or composition either one or both of an improved capacity to hydrolyze a lignocellulosic biomass substrate, in particular one that is mannan-containing, and an improved capacity to reduce viscosity of a biomass substrate mixture, particularly one that is at a high solids level. Such derivatives may be made, for example, to improve expression in a particular host, improve secretion (e.g., by altering the signal sequence), to introduce epitope tags or other sequences that can facilitate the purification and isolation of Tfu Man1 polypeptides. In some embodiments, derivatives may confer more capacity to hydrolyze a lignocellulosic biomass substrate to a cellulase or cellulase and hemicellulase mixture or compostion, as compared to the native Tfu Man1 polypeptide. In some embodiments, derivatives may confer a higher viscosity reduction benefit (e.g., an improvement or even higher speed and/or extent of viscosity reduction) to a cellulase or cellulase and hemicellulase mixture, as compared to the native Tfu Man1 polypeptide.

Tfu Man1 polypeptide derivatives can be prepared by introducing appropriate nucleotide changes into the Tfu Man1 -encoding DNA, or by synthesis of the desired Tfu Man1 polypeptides. Those skilled in the art will appreciate that amino acid changes may alter post-translational processes of the Tfu Man1 polypetpides, such as changing the number or position of glycosylation sites.

Derivatives of the native sequence Tfu Man1 polypeptide or of various domains of the Tfu Man1 described herein can be made, for example, using any of the techniques and guidelines for conservative and non-conservative mutations set forth, for instance, in U.S. Patent No. 5,364,934. Sequence variations may be a substitution, deletion or insertion of one or more codons encoding the Tfu Man1 polypeptide that results in a change in the amino acid sequence of the Tfu Man1 polypeptide as compared with the native sequence Tfu Man1 polypeptide. Optionally, the sequence variation is by substitution of at least one amino acid with any other amino acid in one or more of the domains of the Tfu Man1 polypeptide.

Guidance in determining which amino acid residue may be inserted, substituted or deleted without adversely affecting the desired Tfu Man1 beta-mannanase activity may be found by comparing the sequence of the polypeptide with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of 1 to 5 amino acids. The variation allowed may be determined by systematically making insertions, deletions or substitutions of amino acids in the sequence and testing the resulting derivatives for functional activity using techniques known in the art.

The sequence variations can be made using methods known in the art such as oligonucleotide-mediated (site-directed) mutagenesis, alanine scanning, and PCR mutagenesis. Site-directed mutagenesis (Carter et al., Nucl. Acids Res., 13:4331 (1986); Zoller et al., Nucl. Acids Res., 10:6487 (1987)), cassette mutagenesis (Wells et al., Gene, 34:315 (1985)), restriction selection mutagenesis (Wells et al., Philos. Trans. R. Soc. London SerA, 317:415 (1986)) or other known techniques can be performed on the cloned DNA to produce the Tfu Man1-encoding DNA with a variant sequence.

Scanning amino acid analysis can also be employed to identify one or more amino acids along a contiguous sequence. Among the scanning amino acids the can be employed are relatively small, neutral amino acids. Such amino acids include alanine, glycine, serine, and cysteine. Alanine is often used as a scanning amino acid among this group because it eliminates the side-chain beyond the beta-carbon and is less likely to alter the main-chain conformation of the derivative. Alanine is also often used because it is the most common amino acid. Further, it is frequently found in both buried and exposed positions (Creighton, The Proteins, (W.H. Freeman & Co., N.Y.); Chothia, J. Mol. Biol., 150:1 (1976)). If alanine substitution does not yield adequate amounts of derivative, an isosteric amino acid can be used.

### 5. Anti- Tfu Man1 Antibodies

The present disclosure further describes anti- Tfu Man1 antibodies. Exemplary antibodies include polyclonal and monoclonal antibodies, including chimeric and humanized antibodies.

The anti- Tfu Man1 antibodies of the present disclosure may include polyclonal antibodies. Any convenient method for generating and preparing polyclonal and/or monoclonal antibodies may be employed, a number of which are known to those ordinarily skilled in the art.

Anti- Tfu Man1 antibodies may also be generated using recombinant DNA methods, such as those described in U.S. Patent No. 4,816,567.

The antibodies may be monovalent antibodies, which may be generated by recombinant methods or by the digestion of antibodies to produce fragments thereof, particularly, Fab fragments.

### D. Cell culture media

Generally, the microorganism is cultivated in a cell culture medium suitable for production of the Tfu Man1 polypeptides described herein. The cultivation takes place in a suitable nutrient medium comprising carbon and nitrogen sources and inorganic salts, using procedures and variations known in the art. Suitable culture media, temperature ranges and other conditions for growth and cellulase production are known in the art. As a non-limiting example, a typical temperature range for the production of cellulases by *Trichoderma reesei* is 24°C to 37°C, for example, between 25°C and 30°C.

### a. Cell culture conditions

Materials and methods suitable for the maintenance and growth of fungal cultures are well known in the art. In some aspects, the cells are cultured in a culture medium under conditions permitting the expression of one or more beta-mannanase polypeptides encoded by a nucleic acid inserted into the host cells. Standard cell culture conditions can be used to culture the cells. In some aspects, cells are grown and maintained at an appropriate temperature, gas mixture, and pH. In some aspects, cells are grown at in an appropriate cell medium.

### 6. Compositions Comprising a Recombinant Beta-Mannanase Tfu Man1 Polypeptide

The present disclosure provides engineered enzyme compositions (e.g., cellulase compositions) or fermentation broths enriched with a recombinant Tfu Man1 polypeptides. In some aspects, the composition is a cellulase composition. The cellulase composition can be, *e.g.,* a filamentous fungal cellulase composition, such as a *Trichoderma* cellulase composition. The cellulase composition can be, in some embodiments, an admixture or physical mixture, of various cellulases originating from different microorganisms; or it can be one that is the culture broth of a single engineered microbe co-expressing the celluase genes; or it can be one that is the admixture of one or more individually/separately obtained cellulases with a mixture that is the culture broth of an engineered microbe co-expressing one or more cellulase genes.

In some aspects, the composition is a cell comprising one or more nucleic acids encoding one or more cellulase polypeptides. In some aspects, the composition is a fermentation broth comprising cellulase activity, wherein the broth is capable of converting greater than about 50% by weight of the cellulose present in a biomass sample into sugars. The term "fermentation broth" and "whole broth" as used herein refers to an enzyme preparation produced by fermentation of an engineered microorganism that undergoes no or minimal recovery and/or purification subsequent to fermentation. The fermentation broth can be a fermentation broth of a filamentous fungus, for example, a *Trichoderma, Humicola, Fusarium, Aspergillus, Neurospora, Penicillium, Cephalosporium, Achlya, Podospora, Endothia, Mucor, Cochliobolus, Pyricularia, Myceliophthora* or *Chrysosporium* fermentation broth. In particular, the fermentation broth can be, for example, one of *Trichoderma spp.* such as a *Trichoderma reesei,* or *Penicillium spp.,* such as a *Penicillium funiculosum.* The fermentation broth can also suitably be a cell-free fermentation broth. In one aspect, any of the cellulase, cell, or fermentation broth compositions of the present invention can further comprise one or more hemicellulases.

In some aspects, the whole broth composition is expressed in *T. reesei* or an engineered strain thereof. In some aspects the whole broth is expressed in an integrated strain of *T. reesei* wherein a number of cellulases including a Tfu Man1 polypeptide has been integrated into the genome of the *T. reesei* host cell. In some aspects, one or more components of the polypeptides expressed in the integrated *T. reesei* strain have been deleted.

In some aspects, the whole broth composition is expressed in A. *niger* or an engineered strain thereof.

Alternatively, the recombinant Tfu Man1 polypeptides can be expressed intracellularly. Optionally, after intracellular expression of the enzyme variants, or secretion into the periplasmic space using signal sequences such as those mentioned above, a permeabilisation or lysis step can be used to release the recombinant Tfu Man1 polypeptide into the supernatant. The disruption of the membrane barrier is effected by the use of mechanical means such as ultrasonic waves, pressure treatment (French press), cavitation, or by the use of membrane-digesting enzymes such as lysozyme or enzyme mixtures.

In some aspects, the polynucleotides encoding the recombinant Tfu Man1 polypeptide are expressed using a suitable cell-free expression system. In cell-free systems, the polynucleotide of interest is typically transcribed with the assistance of a promoter, but ligation to form a circular expression vector is optional. In some embodiments, RNA is exogenously added or generated without transcription and translated in cell-free systems.

### 7. Uses of Tfu Man1 Polypeptides to Hydrolyze a Lignocellulosic Biomass Substrate

In some aspects, provided herein are methods for converting lignocelluloses biomass to sugars, the method comprising contacting the biomass substrate with a composition disclosed herein comprising a Tfu Man1 polypeptide in an amount effective to convert the biomass substrate to fermentable sugars. Suitably the biomass substrate comprises GGM and/or GM. In certain embodiments, a suitable biomass substrate may contain up to about 2 wt.% or more, about 3 wt.% or more, about 4 wt.% or more, about 5 wt.% or more, etc. of GGM and/or GM.

In some aspects, the method further comprises pretreating the biomass with acid and/or base and/or mechanical or other physical means In some aspects the acid comprises phosphoric acid. In some aspects, the base comprises sodium hydroxide or ammonia. In some aspects, the mechanical means may include, for example, pulling, pressing, crushing, grinding, and other means of physically breaking down the lignocellulosic biomass into smaller physical forms. Other physical means may also include, for example, using steam or other pressurized fume or vapor to "loosen" the lignocellulosic biomass in order to increase accessibility by the enzymes to the cellulose and hemicellulose. In certain embodiments, the method of pretreatment may also involve enzymes that are capable of breaking down the lignin of the lignocellulosic biomass substrate, such that the accessibility of the enzymes of the biomass hydrolyzing enzyme composition to the cellulose and the hemicelluloses of the biomass is increased.

**Biomass:** The disclosure provides methods and processes for biomass saccharification, using the enzyme compositions of the disclosure, comprising a Tfu Man1 polypeptide. The term "biomass," as used herein, refers to any composition comprising cellulose and/or hemicellulose (optionally also lignin in lignocellulosic biomass materials). Particularly suitable are lignocellulosic biomass materials comprising measureable amounts of galactoglucomannans (GGMs) and/or glucomannan (GMs). Such biomass materials may include, for example, a KRAFT-alkaline pretreated industrial unbleached softwood pulp, FPP-27, which can be obtained from Agence Nationale de la Recherche, France, which contains about 6.5 wt.% mannan; a SPORL-pretreated softwood (Zhu J.Y. et al., (2010) Appl. Microbiol. Biotechnol. 86(5):1355-65; Tian S. et al., (2010) Bioresour. Technol. 101:8678-85), which contains about 4.5 wt.% mannan; spruce, which may contain over 10 wt.% of mannan. As used herein, biomass includes, without limitation, certain softwood trees such as spruce, pine, aspen trees, and wastes derived therefrom, seeds, grains, tubers, plant waste (such as, for example, empty fruit bunches of the palm trees, or palm fibre wastes) or byproducts of food processing or industrial processing (e*.g.,* stalks), corn (including, *e.g.,* cobs, stover, and the like), grasses (including, *e.g.,* Indian grass, such as *Sorghastrum uutaus;* or, switchgrass, *e.g., Panicum* species, such as *Panicum virgatum.*), perennial canes (*e.g.,* giant reeds), wood (including, *e.g*., wood chips, processing waste), paper, pulp, and recycled paper (including, *e.g*., newspaper, printer paper, and the like). Other biomass materials include, without limitation, potatoes, soybean (*e.g*., rapeseed), barley, rye, oats, wheat, beets, and sugar cane bagasse.

The disclosure therefore provides methods of saccharification comprising contacting a composition comprising a biomass material, for example, a material comprising xylan, hemicellulose, and in particular, galactoglucomannans (GGMs) and/or glucomannans (GMs), cellulose, and/or a fermentable sugar, with a Tfu Man1 polypeptide of the disclosure, or a Tfu Man1 polypeptide encoded by a nucleic acid or polynucleotide of the disclosure, or any one of non-naturally occurring the cellulase or cellulase and hemicellulase compositions comprising a Tfu Man1 polypeptide, or products of manufacture of the disclosure.

The saccharified biomass (*e.g.*, lignocellulosic material processed by enzymes of the disclosure) can be made into a number of bio-based products, *via* processes such as, *e.g.,* microbial fermentation and/or chemical synthesis. As used herein, "microbial fermentation" refers to a process of growing and harvesting fermenting microorganisms under suitable conditions. The fermenting microorganism can be any microorganism suitable for use in a desired fermentation process for the production of bio-based products. Suitable fermenting microorganisms include, without limitation, filamentous fungi, yeast, and bacteria. The saccharified biomass can, for example, be made it into a fuel (*e.g.,* a biofuel such as a bioethanol, biobutanol, biomethanol, a biopropanol, a biodiesel, a jet fuel, or the like) *via* fermentation and/or chemical synthesis. The saccharified biomass can, for example, also be made into a commodity chemical (*e.g*., ascorbic acid, isoprene, 1,3-propanediol), lipids, amino acids, polypeptides, and enzymes, *via* fermentation and/or chemical synthesis.

**Pretreatment:** Prior to saccharification or enzymatic hydrolysis and/or fermentation of the fermentable sugars resulting from the saccharifiction, biomass (*e.g*., lignocellulosic material) is preferably subject to one or more pretreatment step(s) in order to render xylan, hemicellulose, cellulose and/or lignin material more accessible or susceptible to the enzymes in the enzymatic composition (for example, the enzymatic composition of the present invention comprising a Tfu Man1 polypeptide) and thus more amenable to hydrolysis by the enzyme(s) and/or the enzyme compositions.

In some aspects, a suitable pretreatment method may involve subjecting biomass material to a catalyst comprising a dilute solution of a strong acid and a metal salt in a reactor. The biomass material can, *e.g.,* be a raw material or a dried material. This pretreatment can lower the activation energy, or the temperature, of cellulose hydrolysis, ultimately allowing higher yields of fermentable sugars. See, *e.g.,* U.S. Patent Nos. 6,660,506; 6,423,145.

In some aspects, a suitable pretreatment method may involve subjecting the biomass material to a first hydrolysis step in an aqueous medium at a temperature and a pressure chosen to effectuate primarily depolymerization of hemicellulose without achieving significant depolymerization of cellulose into glucose. This step yields a slurry in which the liquid aqueous phase contains dissolved monosaccharides resulting from depolymerization of hemicellulose, and a solid phase containing cellulose and lignin. The slurry is then subject to a second hydrolysis step under conditions that allow a major portion of the cellulose to be depolymerized, yielding a liquid aqueous phase containing dissolved/soluble depolymerization products of cellulose. See, *e.g.,* U.S. Patent No. 5,536,325.

In further aspects, a suitable pretreatment method may involve processing a biomass material by one or more stages of dilute acid hydrolysis using about 0.4% to about 2% of a strong acid; followed by treating the unreacted solid lignocellulosic component of the acidhydrolyzed material with alkaline delignification. See, *e.g.,* U.S. Patent No. 6,409,841.

In yet further aspects, a suitable pretreatment method may involve pre-hydrolyzing biomass (*e.g*., lignocellulosic materials) in a pre-hydrolysis reactor; adding an acidic liquid to the solid lignocellulosic material to make a mixture; heating the mixture to reaction temperature; maintaining reaction temperature for a period of time sufficient to fractionate the lignocellulosic material into a solubilized portion containing at least about 20% of the lignin from the lignocellulosic material, and a solid fraction containing cellulose; separating the solubilized portion from the solid fraction, and removing the solubilized portion while at or near reaction temperature; and recovering the solubilized portion. The cellulose in the solid fraction is rendered more amenable to enzymatic digestion. See, *e.g.,* U.S. Patent No. 5,705,369. In a variation of this aspect, the pre-hydrolyzing can alternatively or further involve pre-hydrolysis using enzymes that are, for example, capable of breaking down the lignin of the lignocellulosic biomass material.

In yet further aspects, suitable pretreatments may involve the use of hydrogen peroxide H₂O₂. See Gould, 1984, Biotech, and Bioengr. 26:46-52.

In further aspects, suitable pretreatment of the lignocellulosic biomass materials, in particular those comprising measurable amounts of galactoglucomannans (GGMs) and/or glucomannans (GMs), may include the KRAFT alkaline pretreatment method employed by, for example, the Agence Nationale de la Recherche, France. The KRAFT pretreatment method is a well-known and widely used method to convert wood into wood pulp, typically including the treatment of wood chips with a mixture of sodium hydroxide and sodium sulfide, known in the industry as "white liquor," which breaks down the bonds that link lignin to the cellulose. It is a long-practiced method, mostly in the paper and pulp industry, originally invented by Carl F. Dahl in 1879, as described in U.S. Patent 296,935, issued in 1884. Also included are the SPORL pretreatment method developed by the United States Department of Agriculture specifically for certain softwood biomass feedstocks, for example, for pine, spruce and aspen tree materials, such as described in Zhu et al., (2009) Bioresource Technol. 100:2411-18. The SPORL pretreatment method involves using sulfite to treat wood chips of such softwoods under acidic conditions followed by mechanical size reduction using disk refining. The SPORL method was reported to produce a reduced amount of fermentation inhibitors such as hydroxyl-methyl furfural and/or furfural.

In other aspects, pretreatment can also comprise contacting a biomass material with stoichiometric amounts of sodium hydroxide and ammonium hydroxide at a very low concentration. See Teixeira et al., (1999), Appl. Biochem.and Biotech. 77-79:19-34.

In some embodiments, pretreatment can comprise contacting a lignocellulose with a chemical (*e.g*., a base, such as sodium carbonate or potassium hydroxide) at a pH of about 9 to about 14 at moderate temperature, pressure, and pH. *See* Published International Application WO2004/081185. Ammonia is used, for example, in a preferred pretreatment method. Such a pretreatment method comprises subjecting a biomass material to low ammonia concentration under conditions of high solids. See, *e.g.,* U.S. Patent Publication No. 20070031918 and Published International Application WO 06110901.

### A. The Saccharification Process

In some aspects, provided herein is a saccharification process comprising treating a lignocellulosic biomass material, in particular, one comprising a measurable amount of galactoglucomannans (GGMs) and/or glucomannans (GMs), with an enzyme composition comprising a polypeptide, wherein the polypeptide has beta-mannanase activity and wherein the process results in at least about 50 wt.% (e.g., at least about 55 wt.%, 60 wt.%, 65 wt.%, 70 wt.%, 75 wt.%, or 80 wt.%) conversion of the biomass to fermentable sugars. In some aspects, the biomass comprises lignin. In some aspects the biomass comprises cellulose. In some aspects the biomass comprises hemicelluloses. In some aspects, the biomass comprising cellulose further comprises one or more of mannan, xylan, galactan, and/or arabinan. In certain particular aspects, the biomass comprising cellulose as well as at least a measurable level of galactoglucomannan and/or glucomannan. In some aspects, the biomass may be, without limitation, softwood plants (e.g., pine, spruce, aspen trees), seeds, grains, tubers, plant waste (e.g., empty fruit bunch from palm trees, or palm fibre waste) or byproducts of food processing or industrial processing (*e.g.,* stalks), corn (including, *e.g.,* cobs, stover, and the like), grasses (including, *e.g.,* Indian grass, such as *Sorghastrum nutans;* or, switchgrass, *e.g., Panicum* species, such as *Panicum virgatum*), perennial canes (*e.g.,* giant reeds), woody materials (including, *e.g*., wood chips, processing waste), paper, pulp, and recycled paper (including, *e.g*., newspaper, printer paper, and the like), potatoes, soybean (e.g., rapeseed), barley, rye, oats, wheat, beets, and sugar cane bagasse.

In some aspects, the material comprising biomass is subject to one or more pretreatment methods/steps prior to treatment with the Tfu Man1 polypeptide or the composition comprising the Tfu Man1 polypeptide. In some aspects, the saccharification or enzymatic hydrolysis further comprises treating the biomass with an enzyme composition comprising a Tfu Man1 polypeptide of the invention. The enzyme composition may, for example, comprise one or more cellulases, for example, one or more endoglucanases and optionally also, one or more cellobiohydrolases, and/or one or more beta-glucosidases, in addition to the Tfu Man1 polypeptide. Alternatively, the enzyme composition may comprise one or more other hemicellulases, for example, one or more other beta-mannanases, one or more xylanases, one or more beta-xylosidases, and/or one or more L-arabinofuranosidases. In certain embodiments, the enzyme composition comprises a Tfu Man1 polypeptide of the invention, one or more cellulases and optionally also, one or more other hemicellulases. In some embodiments, the enzyme composition is a fermentation broth composition, optionally subject to some post-production/fermentation processing. In certain embodiments, the enzyme composition is a whole broth formulation.

In some aspects, provided is a saccharification process comprising treating a lignocellulosic biomass material with a composition comprising a polypeptide, wherein the polypeptide has at least about 65% (e.g., at least about 65%, 70%, 75%, 80%, 85%, 90%, 91%, 92%, 93%, 94%, 95%, 96%, 97%, 98%, 99%) sequence identity to SEQ ID NO:2, or to the mature sequence of SEQ ID NO:3, and wherein the process results in at least about 50% (e.g., at least about 55%, 60%, 65%, 70%, 75%, 80%, 85%, or 90%) by weight conversion of biomass to fermentable sugars. In some aspects, lignocellulosic biomass material has been subject to one or more pretreatment methods/steps as described herein.

Other aspects and embodiments of the present compositions and methods will be apparent from the foregoing description and following examples.

### EXAMPLES

The following examples are put forth so as to provide those of ordinary skill in the art with a complete disclosure and description of how to make and use the present compositions and methods, and are not intended to limit the scope of what the inventors regard as their inventive compositions and methods nor are they intended to represent that the experiments below are all or the only experiments performed. Efforts have been made to ensure accuracy with respect to numbers used (e.g. amounts, temperature, etc.) but some experimental errors and deviations should be accounted for.

### EXAMPLE 1

### Cloning of Thermobifida fusca glycosyl hydrolase tfu Man1

The Actinobacterium *Thermobifida fusca* strain YX was selected as a potential source for various glycosyl hydrolases and other enzymes, useful for industrial applications. The nucleic acid sequence of the *tfu Man1* gene encoding the Tfu Man1 (NCBI Accession Number YP_288961) was synthesized by GeneRay Biotech Co., Ltd (Shanghai, China).

TfuMan1 (NCBI accession No.: YP_288961) is a mannan endo-1,4-beta-mannosidase from the Actinobacterium *Thermobifida fusca* YX. The nucleic acid sequence of the gene *tfu Man1* enciding Tfu Man1, is provided herein as SEQ ID NO: 1. The amino acid sequence of the protein encoded by the *tfu Man1* gene is provided herein as SEQ ID NO:2. At the N-terminus, the protein is predicted to have a signal peptide with a length of 28 amino acids as determined by the Signal P 3.0 program (www.cbs.dtu/services/SignalP) set to SignalP-NN system (Emanuelsson et al., Nature Protocols, 2: 953-971, 2007). The presence of a signal sequence suggests that TfuMan1 is a secreted glycosyl hydrolase.

### EXAMPLE 2

### Expression of Thermobifida fusca Tfu Man1 in a Streptomyces lividans host

The synthetic *tfu Man1* gene was digested with *Nhe*I/*BamH*I*,* and ligated into the pKB128 vector digested with the same restriction enzymes to obtain the expression plasmid pML221 (A4-TfuMan1) (**FIGURE 1**)**.** The pKB128 plasmid is a derivative of the pKB105 plasmid (as described in U.S. Pat. Application Publication No. 2006/0154843) and was used as the source of the A4 promoter. The ligation mixture was used to transform chemically competent *E. coli* TOP10 cells (Invitrogen) following the manufacturer's protocol. The transformed cells were plated on Luria Agar plates supplemented with 50 ppm ampicillin and incubated overnight at 37°C. Three transformants were picked from the plate and inoculated into 5 ml Luria Broth supplemented with 50 ppm ampicillin. Cultures were grown overnight at 37°C. The plasmids were extracted, and the sequence of the *tfu Man1* gene was confirmed by DNA sequencing (SEQ ID NO:7). The amino acid sequence of the full length Tfu Man1 expressed from the pML221 plasmid was SEQ ID NO:8, and the mature Tfu Man1 was SEQ ID NO:9.

The pML221 plasmid was used to transform *S*. *lividans* TK23 derived protoplasts, in accordance with the description in U.S. Patent Application Publication No. 2006/0154843. The transformation techniques that were used are described in Kieser et al., (2000) Practical Streptomyces Genetics, The John Innes Foundation, Norwich, United Kingdom. Transformed cells were plated on R5 selection plates and incubated at 30°C for 3 days. One clone from the *Streptomyces* transformation plate was inoculated in TSG medium in shake flasks at 28°C for 3 days. Cultures were then transferred to the *Streptomyces* Modified Medium (as described in U.S. Patent Application Publication No. 2006/0154843), and incubated for an additional 4 days at 28°C. Cells were then harvested by centrifugation, and the supernatant was collected for SDS-PAGE analysis and protein purification, and subsequent characterizations.

The recipe and protocol used for preparing and using the R5 plates are as follows: 206 g sucrose, 0.5 g K2SO4, 20.24 g MgCl2, 20 g glucose, 0.2 g Difco casamino acids, 10 g Difco yeast extracts, 11.46 g TES, 4 g L-Asp, 4 ml of trace elements, 44 g Difco agar, 20 mL 5% K2HPO4, 8 mL 5 M CaCl2•2H2O and 14 mL 1N NaOH were added to a final volume of 1 L after autoclaving. After 20 hours, a layer of thiostrepton (50 µg/mL final concentration) was plated on the top of the plates.

The recipe and protocol used for preparation and using the TSG medium are as follows: 16 g BD Difco tryptone, 4 g BD Bacto soytone, 20 g Sigma caseine (hydrolysate), and 10 g potassium phosphate, dibasic, brought to 1 L. After autoclaving, 50% glucose was added to a final concentration of 1.5%.

### EXAMPLE 3

### Purification of beta-mannanase Tfu Man1 from a culture medium

### of Streptomyces lividans

TfuMan1 was purified using three chromatography steps: (1) a hydrophobic interaction chromatography step; (2) an anion-exchange chromatography step; and (3) a size-exclusion chromatography step. In the first step, ammonium sulphate was added to the 1.6 L of crude broth from the shake flask, to a final concentration of 1 M ammonium sulphate. The pH of the mixture was adjusted to pH 8.0. The mixture was then loaded onto a 50 mL phenyl-sepharose FF column, pre-equilibrated with a 20 mM Tris buffer, pH 8.0, with 1 M ammonium sulphate (buffer A). After washing with the buffer A for 2 column volumes (CVs), the column was eluted with 100%-0 buffer A in 6 CVs, followed by 2 CVs of a 20 mM Tris buffer, pH 8.0 (buffer B) and 3 CVs of MilliQ water. The target polypeptide was eluted from the column in buffer B. The fractions containing target polypeptide were pooled and desalted using ultrafiltration. In the second step, the desalted sample was then loaded onto a 50 mL Q-Sepharose FF column, pre-equilibrated with buffer B. After washing, the column was eluted with a gradient of 0-50% buffer B with 1 M NaCl in 8 CVs. The target polypeptide was eluted at about 250 mM NaCl. The fractions containing target polypeptide were again pooled and concentrated to a volume of less than 10 mL. In the third step, the concentrated fraction pool was applied to a Superdex 75 XK 26X60 column, pre-equilibrated in a 20 mM Na-phosphate buffer, pH 7 with 0.15 M NaCl. The fractions from this column containing the target polypeptide were collected, pooled and concentrated using 10 KDa Amicon Ultra-15 devices.

The purity of the polypeptide was determined using SDS-PAGE, and the predicted molecular weight of the Tfu Man1 polypeptide, which has 425 amino acid residues and has an estimated molecular weight of about 45 kDa, was used to confirm the identity of the Tfu Man1 polypeptide. The purified Tfu Man1 polypeptide was used to perform the pH profile, temperature profile and thermostability profile studies below.

### EXAMPLE 4

### Expression of Thermobifida fusca Tfu Man1 in a T. reesei host

The *tfu Man1* gene can be amplified from *Thermobifida fusca* genomic DNA using PCR, with the native signal sequence and a CACC sequence added to the 5' end of the forward primer for directional Gateway cloning (Invitrogen, Carlsbad, CA). Alternatively, a *T. reesei cbhI* signal sequence might be employed, substituting for the native signal sequence. The PCR product of the *tfu Man1* gene can be purified using a Qiaquick PCR Purification Kit (Qiagen). The purified PCR product can then be cloned into the pENTR/D-TOPO vector, transformed into *One Shot® TOP10* Chemically Competent *E. coli* cells (Invitrogen), and then plated onto LA plates containing 50 ppm kanamycin. Plasmid DNA can then be obtained from the *E. coli* transformants, using a QIAspin plasmid preparation kit (Qiagen).

The nucleotide sequence of the inserted DNA can then be confirmed as SEQ ID NO:1 using well-known sequencing methods. The pENTR/D-TOPO_*tfuMan1* vector including the confirmed *tfu Man1* gene sequence can then be recombined with the expression vector pTrex3gM (*see, e.g.,* International Published Patent Application WO 05/001036, FIGURE 2), using an LR clonase® reaction (*see,* protocols by Invitrogen).

The product of the LR clonase® reaction (i.e., the vector pTrex3gM_TfuMan1) can then be transformed into *E. coli One Shot*® *TOP10* Chemically Competent cells (Invitrogen) and plated on LA medium containing 50 ppm carbenicillin. The pTrex3gM vector also contains the *Aspergillus tubingensis amdS* gene, encoding acetamidase, as a selectable marker for transformation of *T. reesei.* The pTrex3gM vector further contains a *cbhI* promoter and terminator, which flank the *tfu Man1* sequence.

Thereafter, about 0.5 to 1 µg of the expression vector pTrex3gM_TfuMan1 (or a fragment amplified by PCR) can be used to transform a *T. reesei* strain with its major cellulase genes deleted, for example, a six-fold deletion strain as described in, *e.g.,* in International Patent Application Publication No. WO 2010/141779), using the PEG-protoplast method with modifications as described herein.

For protoplast preparation, spores can be grown for 16-24 hours at 24°C in a *Trichoderma* Minimal Medium MM, containing 20 g/L glucose, 15 g/L KH₂PO₄, pH 4.5, 5 g/L (NH₄)₂SO₄ 0.6 g/L MgSO₄x7H₂O, 0.6 g/L CaCl₂x2H₂O, 1 mL of 1000 X *T. reesei* Trace elements solution (5 g/L FeSO₄x7H₂O, 1.4 g/L ZnSO₄x7H₂O, 1.6 g/L MnSO₄ x H₂O, 3.7 g/L CoCl₂ x 6H₂O) with shaking at 150 rpm. Germinating spores can then be harvested by centrifugation and treated with 50 mg/mL of Glucanex G200 (Novozymes AG) solution to lyse the fungal cell walls. Further preparation of the protoplasts can be performed in accordance with a method described by Penttilä et al. Gene 61(1987)155-164. The transformation mixture, containing about 1 µg of DNA and at least 1 x 10⁷ protoplasts in a total volume of 200 µL, can then be treated with 2 mL of 25% PEG solution, diluted with 2 volumes of 1.2 M sorbitol/10 mM Tris, pH7.5, 10 mM CaCl₂, mixed with 3% selective top agarose MM containing 20 mM acetamide. The resulting mixture is then poured onto 2% selective agarose plate containing acetamide. Followed by that, plates are incubated for 7-10 days at 28°C. Single transformants are then transferred onto fresh MM plates containing acetamide. Spores from independent clones are then used to inoculate a fermentation medium in either 96-well microtiter plates or shake flasks.

Secreted protein from the culture broths can be purified, optionally subject to some post-fermentation processing, or can be used directly for saccharification or hydrolyzing mannan-containing lignocellulosic biomass substrates

### EXAMPLE 5

### The beta-mannanase activity of Tfu Man1

The beta-1,4 mannanase activity of Tfu Man1 was measured using 1% galactomannan (Carob; Low Viscosity) (P-GALML; Lot 10501) purchased from Megazyme International Ireland (Bray, Ireland) as a substrate. The assay was performed in a 50 mM sodium acetate buffer, pH 5.0, containing 0.005% Tween-80, whereby the polypeptide and the substrate were incubated at 50°C for 10 minutes. Alternatively the assay was performed in a 50 mM HEPES buffer, pH 8.2, containing 0.005% Tween-80, whereby the polypeptide and the substrate were incubated at 30°C for 30 minutes.

The reducing sugar(s) released from the hydrolysis reaction was quantified using a PAHBAH (*p*-Hydroxy benzoic acid hydrazide) assay as described by Lever (1972) Anal. Biochem. 47:248. A standard curve was prepared using various amounts of mannose as standards, and the specific enzyme activity units were calculated. Specifically one mannanase unit was defined as the amount of enzyme required to generate 1 micromole of mannose reducing sugar equivalents per minute under a given set of conditions.

As measured, the specific activity of the purified Tfu Man1 polypeptide was about 23 units/mg at pH 5.0, and about 5 units/mg at pH 8.2.

### EXAMPLE 6

### The pH Profile of Tfu Man1

The pH profile of Tfu Man1 was determined using locust bean gum from *Ceratonia siliqua seeds* (Sigma G0753) as substrate. Activity assays were performed in a sodium citrate/sodium phosphate buffer, having various pH values in a range between pH 2 and pH 9. Twenty five (25) µL of a 0.5 M sodium citrate/sodium phosphate buffer was added to 65 µL of locust bean gum (1% aqueous solution) in a 96-well plate, and the substrate was equilibrated at the assay temperature of 50°C prior to the addition of enzyme. After carrying on for 10 minutes, the enzyme reaction was stopped by transferring 10 µL of the reaction mixture to a 96-well PCR plate well, which contained100 µL of PAHBAH solution. The PCR plate was then incubated at 95°C for 5 minutes in a Bio-Rad DNA Engine. The PCR plate was subsequently cooled on ice and 100 µL of the mixture in the well was transferred to a new 96-well assay plate.

The amount of reducing sugar(s) released from the substrate was determined by measuring the optical density of the reaction mixture following the completion of the reaction as described above at 410 nm in a spectrophotometer. The enzyme activity at each pH was reported as relative activity where the activity at the pH optimum was normalized to 100%.

The pH profile of Tfu Man1 is shown in **FIGURE 3****.** Tfu Man1 was found to have an optimum pH at about pH 5.0. The polypeptide was also found to retain greater than 70% of its maximum activity between pH 4.2 and pH 7.8.

### EXAMPLE 7

### The Temperature Profile of Tfu Man1

The temperature optimum of purified Tfu Man1 polypeptide was determined by measuring the beta-mannanase of Tfu Man1, at various temperatures between 30°C and 78°C, in a 50 mM sodium citrate buffer, pH 6.0, for 10 minutes. The activity was reported as relative activity where the activity at the temperature optimum was normalized to 100%. The temperature profile of Tfu Man1 is shown in **FIGURE 4****.**

Tfu Man1 was found to have an optimum temperature of about 71°C. Tfu Man1 was also found to retain greater than 80% of its maximum activity between the temperatures of 58°C and 78°C.

### EXAMPLE 8

### The Thermostability Profile of Tfu Man1

The thermostability of Tfu Man1 was determined in a 50 mM sodium citrate buffer, pH 6.0. The enzyme was incubated in a PCR thermal cycler at the desired temperature for 2 hours. The remaining or residual activity of each sample was measured as described in Example 5 above. The activity of a control Tfu Man1 sample kept on ice was used to define a 100%-retained activity. The thermostability profile of Tfu Man 1 is shown in **FIGURE 5****.**

Tfu Man1 retained about 50% activity over a 2-hour incubation period at 72°C. No activity loss was detected after a 2-hour incubation period at temperatures lower than 60 °C, indicating that Tfu Man1 was remarkably thermostable.

### EXAMPLE 9

### Hydrolysis of an alkaline KRAFT-pretreated softwood biomass substrate using an enzyme composition comprising Tfu Man1.

An alkaline KRAFT-pretreated softwood substrate FPP-27 was obtained from Agence Nationale de la Recherche, France (ARN-05-BIOE-007) through a research project funded by L'Agence Nationale de I'Environmental et de la Maitrise de I'Energie (ADEME 0501 C0099), and a composition analysis was conducted, indicating the following content of the biomass: ∼2.5 wt.% Klason lignin; -81.4 wt.% glycan; ∼ 7.9 wt.% xylan, ∼0.8 wt.% galactan; and ∼6.5 wt.% mannan. The substrate, in an amount of 1.93 g, at a dry solids loading level of 8.6% and total cellulose loading of 7% was mixed with an Accellerase® TRIO™ sample (which was pre-diluted into the desired concentration, as needed, using 0.05 M sodium citrate buffer, pH 5.0) at 10 mg/g glucan into a reaction mixture as a control. The substrate, in an amount of 1.93 g, at the same dry solids loading level of 8.6% and total cellulose loading of 7%, was mixed with a blended enzyme having 9 mg/g glucan of Accellerase® TRIO™ and 1 mg/g glucan of Tfu Man1, 1 mg/g glucan of ScoMan1, or 1 mg/g glucan of Bsp Man1, or 1 mg/g glucan of Msp Man2, in a reaction mixture. The reaction mixtures and the control mixture were adjusted to pH 5 using a 0.1 M sodium citrate buffer. A 5% sodium azide was added to each of the reaction mixtures and control mixture to control microbial growth.

The reaction mixture and the control mixture are then incubated in a New Brunswick Scientific Innova 44 Incubator Shaker at 50°C, with gentle agitation at 200 rpm. After 24 hours, 48 hours, 72 hours, a small sample of about 200 µL was taken from each of the reaction mixture, diluted in 200 µL of MilliQ water, followed by filtration through a 0.2 µm filter. The filtrate was then injected into a Waters HPLC, equipped with a Waters 2695 Separation Module, set at a flow rate of 0.6 mL/min, and a mobile phase of MilliQ water degassed with 0.2 µm filter; a Phenomenex Rezex RCM 300 x 7.8 mm column, and in tandem, an RPM 300 x 7.8 mm column; a Phenomenex Security Guard Kit, including a Carbo-Ca 4 x 3.0 mm security guard cartridge; and a Waters 2414 Refractive Index Detector, set at an operating temperature of 50°C. The reaction mixtures as well as the control sample were analyzed for the amount of glucose, xylose and mannose. The results are presented in **FIGURES 6A-6C****.**

The reaction mixtures were allowed to continue for as long as 72 hours, and the total carbohydrate conversion during the time period of 24-72 hours of each of the samples were plotted and presented as time courses in **FIGURE 7****.**

### EXAMPLE 10

### Viscosity reduction by Tfu Man1

An FPP-27 KRAFT-pretreated softwood pulp can be used, which has been determined via a composition analysis to contain the following: ∼2.5 wt.% Klason lignin; ∼81.4 wt.% glycan; ∼ 7.9 wt.% xylan, ∼0.8 wt.% galactan; and ∼6.5 wt.% mannan. Alternatively the same SPORL-pretreated softwood substrate can be used, which has been determined by a composition analysis to contain the following: ∼32.4 wt.% klason lignin; ∼ 49.4 wt.% glucan; ∼3.4 wt.% xylan; and ∼4.6 wt.% mannan. As a control substrate, an acid-pretreated whole hydrolysate corn stover (whPCS) (*see, e.g.,* www.nrel.gov/ docs/fyllosti/47764.pdf), which does not contain any GGM or GM, but contains ∼ 33.8 wt.% glucan, no xylan, and ∼ 2.2 wt.% galactan, can be used.

An amount of 1.93 g of such a substrate (including, for example the FPP-27 substrate or the SPORL-pretreated softwood substrate, and the control whPCS substrate), at a dry solids loading level of 8.6% and a total glucan loading of 7.0%, can then be mixed with 10 mg/g glucan of Accellerase® TRIO™ as a control mixture, and with 1 mg/g glucan of Tfu Man1 plus 9 mg/g glucan of Accellerase® TRIO™ in a reaction mixture. The reaction mixture and the control mixture are then adjusted to pH 5.0 using a 0.1 M sodium citrate buffer, and incubation can take place with gentle agitation at a temperature of about 50°C, for at least 16 hours.

After at least 16 hours of incubation, the viscosity of each of the resulting mixtures (about 2-3 grams of sample) can be determined using the Rapid Visco Analyzer Super 4 Viscometer. (Newport Scientific). The Tfu Man1 polypeptide, when mixed with Accellerase® TRIO™ in the above-described proportions, impart a substantial viscosity reduction benefit, such as, for example, achieving at least a 20% reduced viscosity at the 16-hour incubation point.

## Claims

1. An enzyme composition comprising a recombinant polypeptide comprising an amino acid sequence that is at least 65% identical to the amino acid sequence of SEQ ID NO:2 or SEQ ID NO:3, wherein the polypeptide has beta-mannanase activity, and one or more cellulases.

2. The enzyme composition of claim 1, wherein the recombinant polypeptide improves the hydrolysis performance of a cellulase composition when the polypeptide constitutes up to 20 wt.% of the cellulase composition, wherein the improved hydrolysis performance comprises:
(a) an increased % glucan conversion, an increased % xylan conversion, and/or an increased % glucan and % xylan conversion from a given lignocellulosic biomass substrate under the same hydrolysis conditions; or
(b) an at least about 5% faster viscosity reduction of a given lignocellulosic biomass substrate under the same hydrolysis conditions.

3. The enzyme composition of claim 1 or 2, wherein the recombinant polypeptide has an increased beta-mannanase activity as compared to the beta-mannanase activity of:
(i) ScoMan1 comprising SEQ ID NO:4;
(ii) Bsp Man1 comprising SEQ ID NO:5; or
(iii) Msp Man2 comprising SEQ ID NO:6.

4. The enzyme composition of any one of claims 1-3, wherein the recombinant polypeptide retains greater than 70% of its maximum beta-mannanase activity when incubated at a pH range from pH 4.2 to pH 7.8.

5. The enzyme composition of any one of claims 1-4, wherein the recombinant polypeptide has optimum beta-mannanase activity at a pH of about 5.0.

6. The enzyme composition of any one of claim 1-5, wherein the recombinant polypeptide retains at least 80% or more of its maximum beta-mannanase activity when incubated at a temperature of between 58°C and 78°C.

7. The enzyme composition of any one of claims 1-6, wherein the recombinant polypeptide has optimum beta-mannanase activity at a temperature of about 71°C.

8. The enzyme composition of any one of claims 1-7, wherein the recombinant polypeptide retains at least 50% of its maximum beta-mannanase activity when incubated for about 2 hours at a temperature of about 72°C.

9. The enzyme composition of any one of claims 1-8, wherein the recombinant polypeptide retains at least 99% of its maximum beta-mannanase activity when incubated for about 2 hours at a temperature of up to 60 °C.

10. The enzyme composition of any one of claims 1-9, wherein the recombinant polypeptide comprises an amino acid sequence that is at least 70%, at least 75%, at least 80%, or at least 90% identical to the amino acid sequence of SEQ ID NO:2 or SEQ ID NO:3.

11. The enzyme composition of any one of the preceding claims, wherein the one or more cellulases are selected from one or more beta-glucosidases, one or more cellobiohydrolases, and one or more endoglucanases.

12. The enzyme composition of any one of claims 1-11, further comprising one or more other hemicellulases.

13. The enzyme composition of claims 12, wherein the one or more other hemicellulases are selected from one or more other beta-mannanases, one or more one or more xylanases, one or more beta-xylosidases, and one or more L-arabinofuranosidases.

14. A method for hydrolyzing a lignocellulosic biomass substrate, comprising:
contacting the lignocellulosic biomass substrate with the enzyme composition of any one of claims 1-13, to yield glucose and other sugars.

15. A composition comprising the enzyme composition of any one of claims 1-13 and a lignocellulosic biomass substrate.

16. The method of claim 14 or the composition of claim 15, wherein the lignocellulosic biomass substrate comprises up to about 20 wt.%, or up to about 15 wt.%, or up to about 10 wt.% of galactoglucomannan and/or glucomannan.

## Patentansprüche

1. Enzym-Zusammensetzung, umfassend ein rekombinantes Polypeptid, das eine Aminsosäuresequenz aufweist, die zu mindestens 65% identisch ist mit der Aminsosäuresequenz von SEQ ID NO: 2 oder SEQ ID NO: 3, wobei das Polypeptid über Beta-Mannase-Aktivität und über eine oder mehrere Cellulasen verfiigt.

2. Enzym-Zusammensetzung nach Anspruch 1, wobei das rekombinante Polypeptid die Hydrolyseleistung einer Cellulase-Zusammensetzung verbessert, wenn das Polypeptid bis zu 20 Gewichtsprozent der Cellulase-Zusammensetzung ausmacht, wobei die verbesserte Hydrolyseleistung umfasst:
(a) eine erhöhte prozentuale Umsetzung von Glucan, eine erhöhte prozentuale Umsetzung von Xylan, und/oder eine erhöhte prozentuale Umsetzung von Glucan und Xylan für ein vorgegebenes Substrat von Lignocellulose enthaltender Biomasse unter den gleichen Bedingungen der Hydrolyse; oder
(b) eine mindestens etwa 5 Prozent schnellere Verringerung der Viskosität eines gegebenen Substrats von Lignocellulose enthaltender Biomasse unter den gleichen Bedingungen der Hydrolyse.

3. Enzym-Zusammensetzung nach Anspruch 1 oder 2, wobei das rekombinante Polypeptid über eine erhöhte Beta-Mannase-Aktivität verfügt, und zwar im Vergleich zu der Beta-Mannase-Aktivität von:
(i) SEQ ID NO: 4 aufweisendem ScoMan1;
(ii) SEQ ID NO: 5 aufweisendem Bsp Man1; oder
(iii) SEQ ID NO: 6 aufweisendem Msp Man2.

4. Enzym-Zusammensetzung nach einem der Ansprüche 1 bis 3, wobei das rekombinante Polypeptid mehr als 70% seiner maximalen Beta-Mannase-Aktivität bewahrt, wenn es in einem pH-Wertbereich von pH 4,2 bis pH 7,8 inkubiert wird.

5. Enzym-Zusammensetzung nach einem der Ansprüche 1 bis 4, wobei das rekombinante Polypeptid ein Optimum der Beta-Mannase-Aktivität bei einem pH-Wert von etwa 5,0 hat.

6. Enzym-Zusammensetzung nach einem der Ansprüche 1 bis 5, wobei das rekombinante Polypeptid mindestens 80% oder mehr von seiner maximalen Beta-Mannase-Aktivität bewahrt, wenn es bei einer Temperatur zwischen 58 °C und 78 °C inkubiert wird.

7. Enzym-Zusammensetzung nach einem der Ansprüche 1 bis 6, wobei das rekombinante Polypeptid ein Optimum der Beta-Mannase-Aktivität bei einer Temperatur von etwa 71 °C hat.

8. Enzym-Zusammensetzung nach einem der Ansprüche 1 bis 7, wobei das rekombinante Polypeptid mindestens 50% seiner maximalen Beta-Mannase-Aktivität bewahrt, wenn es etwa zwei Stunden bei einer Temperatur von etwa 72 °C inkubiert wird.

9. Enzym-Zusammensetzung nach einem der Ansprüche 1 bis 8, wobei das rekombinante Polypeptid mindestens 99% seiner maximalen Beta-Mannase-Aktivität bewahrt, wenn es etwa zwei Stunden bei einer Temperatur von bis zu 60 °C inkubiert wird.

10. Enzym-Zusammensetzung nach einem der Ansprüche 1 bis 9, wobei das rekombinante Polypeptid eine Aminsosäuresequenz aufweist, die zu mindestens 70%, zu mindestens 75%, zu mindestens 80% oder zu mindestens 90% mit der SEQ ID NO: 2 oder SEQ ID NO: 3 identisch ist.

11. Enzym-Zusammensetzung nach einem der vorgenannten Ansprüche, wobei die eine oder die mehreren Cellulasen ausgewählt sind aus einer oder mehreren Beta-Glucosidasen, einer oder mehreren Cellobiohydrolasen und einer oder mehreren Endoglucanasen.

12. Enzym-Zusammensetzung nach einem der Ansprüche 1 bis 11, ferner umfassend eine oder mehrere andere Hemicellulasen.

13. Enzym-Zusammensetzung nach Anspruch 12, wobei die eine oder mehreren anderen Hemicellulasen ausgewählt sind aus einer oder mehreren anderen Beta-Mannasen, einer oder mehreren Xylanasen, einer oder mehreren Beta-Xylosidasen und einer oder mehreren L-Arabino-Furanosidasen.

14. Verfahren zum Hydrolysieren eines Substrats von Lignocellulose enthaltender Biomasse, umfassend:
Inkontaktbringen des Substrats von Lignocellulose enthaltender Biomasse mit der Enzym-Zusammensetzung nach einem der Ansprüche 1 bis 13, um Glucose und andere Zuckerstoffe zu erhalten.

15. Zusammensetzung, umfassend die Enzym-Zusammensetzung nach einem der Ansprüche 1 bis 13 und ein Substrat von Lignocellulose enthaltender Biomasse.

16. Verfahren nach Anspruch 14 oder Zusammensetzung nach Anspruch 15, wobei das Substrat von Lignocellulose enthaltender Biomasse bis zu etwa 2 Gewichtsprozent oder bis zu etwa 15 Gewichtsprozent oder bis zu etwa 10 Gewichtsprozent Glactoglucomannan und/oder Glucomannan aufweist.

## Revendications

1. Composition d'enzyme comprenant un polypeptide recombinant comprenant une séquence d'acides aminés qui est au moins à 65% identique à la séquence d'acides aminés de la SEQ ID NO: 2 ou de la SEQ ID NO: 3, dans laquelle le polypeptide a une activité de bêta-mannanase, et une ou plusieurs cellulases.

2. Composition d'enzyme selon la revendication 1, dans laquelle le polypeptide recombinant améliore la performance à l'hydrolyse d'une composition de cellulase lorsque le polypeptide constitue jusqu'à 20% en poids de la composition de cellulase, laquelle performance d'hydrolyse améliorée comprend:
(a) un % de conversion de glucane accru, un % de conversion de xylane accru et/ou un % de conversion de glucane et un % de conversion de xylane accrus à partir d'un substrat de biomasse lignocellulosique donné dans les mêmes conditions d'hydrolyse; ou
(b) un % de réduction de viscosité au moins environ 5% plus rapide d'un substrat de biomasse lignocellulosique donné dans les mêmes conditions d'hydrolyse.

3. Composition d'enzyme selon la revendication 1 ou 2, dans laquelle le polypeptide recombinant a une activité de bêta-mannanase accrue par rapport à l'activité de bêta-mannanase de:
(i) ScoMan1 comprenant la SEQ ID NO: 4;
(ii) Bsp Man 1 comprenant la SEQ ID NO: 5; ou
(iii) Msp Man2 comprenant la SEQ ID NO: 6.

4. Composition d'enzyme selon l'une quelconque des revendications 1 à 3, dans laquelle le polypeptide recombinant retient plus de 70% de son activité de bêta-mannanase maximale lorsqu'il est incubé à une gamme de pH de pH 4,2 à pH 7,8.

5. Composition d'enzyme selon l'une quelconque des revendications 1 à 4, dans laquelle le polypeptide recombinant a une activité de bêta-mannanase optimale à un pH d'environ 5,0.

6. Composition d'enzyme selon l'une quelconque des revendications 1 à 5, dans laquelle le polypeptide recombinant retient au moins 80% ou plus de son activité de bêta-mannanase maximale lorsqu'il est incubé à une température d'entre 58°C et 78°C.

7. Composition d'enzyme selon l'une quelconque des revendications 1 à 6, dans laquelle le polypeptide recombinant a une activité de bêta-mannanase optimale à une température d'environ 71°C.

8. Composition d'enzyme selon l'une quelconque des revendications 1 à 7, dans laquelle le polypeptide recombinant retient au moins 50% de son activité de bêta-mannanase maximale lorsqu'il est incubé pendant environ 2 heures à une température d'environ 72°C.

9. Composition d'enzyme selon l'une quelconque des revendications 1 à 8, dans laquelle le polypeptide recombinant retient au moins 99% de son activité de bêta-mannanase maximale lorsqu'il est incubé pendant environ 2 heures à une température de jusqu'à 60°C.

10. Composition d'enzyme selon l'une quelconque des revendications 1 à 9, dans laquelle le polypeptide recombinant comprend une séquence d'acides aminés qui est au moins à 70%, au moins à 75%, au moins à 80% ou au moins à 90% identique à la séquence d'acides aminés de la SEQ ID NO: 2 ou de la SEQ ID NO: 3.

11. Composition d'enzyme selon l'une quelconque des revendications précédentes, dans laquelle lesdites une ou plusieurs cellulases est (sont) choisie(s) parmi une ou plusieurs bêta-glucosidases, une ou plusieurs cellobiohydrolases et une ou plusieurs endoglucanases.

12. Composition d'enzyme selon l'une quelconque des revendications 1 à 11, comprenant en outre une ou plusieurs autres hémicellulases.

13. Composition d'enzyme selon la revendication 12, dans laquelle lesdites une ou plusieurs autres hémicellulases est (sont) choisie(s) parmi une ou plusieurs autres bêta-mannanases, une ou plusieurs xylanases, une ou plusieurs bêta-xylosidases et une ou plusieurs L-arabinofuranosidases.

14. Procédé pour hydrolyser un substrat de biomasse lignocellulosique, comprenant:
la mise en contact du substrat de biomasse lignocellulosique avec la composition d'enzyme selon l'une quelconque des revendications 1 à 13, pour donner du glucose et d'autres sucres.

15. Composition comprenant la composition d'enzyme selon l'une quelconque des revendications 1 à 13 et un substrat de biomasse lignocellulosique.

16. Procédé selon la revendication 14 ou composition selon la revendication 15, dans lesquels le substrat de biomasse lignocellulosique comprend jusqu'à environ 20% en poids, ou jusqu'à environ 15% en poids ou jusqu'à environ 10% en poids de galactoglucomannane et/ou de glucomannane.
